(19) Europäisches Patentamt
European Patent Office
Office européen des brevets

(11) **EP 3 948 877 B1**

(12) **EUROPEAN PATENT SPECIFICATION**

(45) Date of publication and mention
of the grant of the patent:
**15.05.2024 Bulletin 2024/20**

(21) Application number: **20718779.0**

(22) Date of filing: **30.03.2020**

(51) International Patent Classification (IPC):
**G16C 20/30** (2019.01)    **G16C 60/00** (2019.01)

(52) Cooperative Patent Classification (CPC):
**G16C 20/30;** G16C 60/00

(86) International application number:
**PCT/IB2020/053017**

(87) International publication number:
**WO 2020/194281 (01.10.2020 Gazette 2020/40)**

(54) **CONFIGURATIONAL ENERGY CALCULATION AND CRYSTAL STRUCTURE PREDICTION**

KONFIGURIERBARE ENERGIEBERECHNUNG UND KRISTALLSTRUKTURVORHERSAGE

CALCUL D'ÉNERGIE DE CONFIGURATION ET PRÉDICTION DE STRUCTURE CRISTALLINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.03.2019 GB 201904340**

(43) Date of publication of application:
**09.02.2022 Bulletin 2022/06**

(60) Divisional application:
**23208037.4**

(73) Proprietor: **University College Dublin,
National University of Ireland
Dublin 4 (IE)**

(72) Inventors:
• **BURNHAM, Christian
Dublin 4 (IE)**
• **ENGLISH, Niall
Dublin 4 (IE)**

(74) Representative: **Sonnenberg Harrison
Partnerschaft mbB
Herzogspitalstraße 10a
80331 München (DE)**

(56) References cited:
• **CHRISTIAN J. BURNHAM ET AL: "Crystal
Structure Prediction via Basin-Hopping Global
Optimization Employing Tiny Periodic Simulation
Cells, with Application to Water-Ice", JOURNAL
OF CHEMICAL THEORY AND COMPUTATION:
JCTC, vol. 15, no. 6, 14 May 2019 (2019-05-14),
pages 3889-3900, XP055713890, US ISSN:
1549-9618, DOI: 10.1021/acs.jctc.9b00073**
• **RAFAL PODESZWA ET AL: "Crystal structure
prediction for cyclotrimethylene trinitramine
(RDX) from first principles", PHYSICAL
CHEMISTRY CHEMICAL PHYSICS, vol. 11, no. 26,
28 April 2009 (2009-04-28), pages 5512-5518,
XP055714870, ISSN: 1463-9076, DOI:
10.1039/b902015b**
• **B. R. BROOKS ET AL: "CHARMM: The
biomolecular simulation program : CHARMM:
The Biomolecular Simulation Program",
JOURNAL OF COMPUTATIONAL CHEMISTRY.,
vol. 30, no. 10, 14 May 2009 (2009-05-14), pages
1545-1614, XP055715280, GB ISSN: 0192-8651,
DOI: 10.1002/jcc.21287**
• **MIN JI ET AL: "Comparing efficiencies of genetic
and minima hopping algorithms for crystal
structure prediction", PHYSICAL CHEMISTRY
CHEMICAL PHYSICS, vol. 12, no. 37, 1 January
2010 (2010-01-01), pages 11617-11623,
XP055715704, ISSN: 1463-9076, DOI:
10.1039/c004096g**

**(Cont. next page)**

EP 3 948 877 B1

- **VERWER P ET AL: "COMPUTER SIMULATION TO PREDICT POSSIBLE CRYSTAL POLYMORPHS",** REVIEWS IN COMPUTATIONAL CHEMISTRY, VCH, NEW YORK, NY, US, vol. 12, 1 October 1998 (1998-10-01), pages 327-365, XP008042156, ISSN: 1069-3599
- **V. BUCH ET AL: "Simulations of H 2 O Solid, Liquid, and Clusters, with an Emphasis on Ferroelectric Ordering Transition in Hexagonal Ice",** JOURNAL OF PHYSICAL CHEMISTRY PART B, vol. 102, no. 44, 1 October 1998 (1998-10-01), pages 8641-8653, XP055713867, US ISSN: 1520-6106, DOI: 10.1021/jp980866f
- **V. BUCH ET AL: "A new molecular-dynamics based approach for molecular crystal structure search",** JOURNAL OF CHEMICAL PHYSICS, vol. 123, no. 5, 1 August 2005 (2005-08-01), page 051108, XP055713857, US ISSN: 0021-9606, DOI: 10.1063/1.2000230
- **HONRAO SHREYAS ET AL: "Machine learning of ab-initio energy landscapes for crystal structure predictions",** COMPUTATIONAL MATERIALS SCIENCE, vol. 158, 28 November 2018 (2018-11-28), pages 414-419, XP085581258, ISSN: 0927-0256, DOI: 10.1016/J.COMMATSCI.2018.08.041
- **Qiang Zhu ET AL: "Crystal Structure Prediction and Its Application in Earth and Materials Sciences" In: "TOPICS IN CURRENT CHEMISTRY.",** 1 January 2014 (2014-01-01), SPRINGER, BERLIN., DE, XP055618384, ISSN: 0340-1022 vol. 345, pages 223-256, DOI: 10.1007/128_2013_508, the whole document
- **PRICE S L: "From crystal structure prediction to polymorph prediction: interpreting the crystal energy landscape",** PHYSICAL CHEMISTRY CHEMICAL PHYSICS,, vol. 10, no. 15, 21 April 2008 (2008-04-21), pages 1996-2009, XP008108430, ISSN: 1463-9076, DOI: 10.1039/B719351C

**Description**

**Field of Invention**

The current invention relates to a method for determining one or more stable crystal structures using a computer-implemented method, thereby determining potential properties of the one or more stable crystal structures, and thereby controlling the necessary parameters for manufacturing the one or more stable crystal structures.

**Background of the Invention**

Crystal-structure prediction (CSP) is fundamental in understanding the behaviour of materials. Precise knowledge of the crystal structure permits calculation of the material's physical and chemical properties under different environmental conditions. The latter opens up the possibility to design materials to suit a wide number of applications, including for structured based drug design or bio-materials genomics.

CSP can be thought of as an optimisation problem, where the enthalpy of the system is the quantity to be optimised; the most stable crystal structure is that with the minimum enthalpy. Experimental methods such as X-ray diffraction are often used to characterise the crystal structure, but cannot be used to predict it *per se.* Additionally, in some instances, experimental data may be of unable to determine crystal structure. For example, this might be due to defective samples when subjected to high pressures or temperatures, and in such cases theoretical methods provide the only solution to predict crystal structure (for example dangerous or toxic environments making experimental determination more difficult).

A real crystal comprises too many molecules to realistically simulate. Fortunately, however, there are a number of well-established simulation methods that can be employed for systems with periodic boundary conditions. Such methods search for an enthalpy minimum of the system with respect to the coordinates in one simulation cell, which it is assumed are then replicated to describe an infinite crystal. Thus, the general approach is to use optimisation algorithms which search the configurational space of nuclear coordinates inside one simulation cell, trying to locate structures with the lowest enthalpy.

To predict the crystal structure, local-optimisation methods are often employed, with crystal structures iterated to find energy minima. However, the number of minima increases dramatically with the system size, and for even moderately sized systems, there are typically too many minima to have a realistic chance of locating the lowest ones using such local-optimisation methods. Therefore, there has been much study of more 'global' optimisation approaches, which attempt to use strategies to not just locate local minima, but to more intelligently search the space for the lowest lying minima, and in the best case, to locate the global minimum.

There exists many computational techniques for CSP, but none of them tend to match basin-hopping (BH) in terms of universality and versatility in locating global crystal-structure minima. However, this technique often requires inputting some external data, for example, in the form of experimentally derived lattice vectors or densities. More importantly, predicting crystal structure with such conventional techniques becomes problematic when applied to materials possessing very small unit cells (or simulation cells). Applying this technique to such materials often leads to truncation of the non-electrostatic part of the interaction between particles, leading to inaccurate results. One solution would be to define large enough simulation cells, however, this since the number of minima scales dramatically with the system size, there will be a notable increase in the computational cost.

In conventional simulation methods the cut-off radius, which defines a non-electrostatic interaction potential cut-off distance between particles in a crystalline solid, is restricted to the unit cell of the crystal structure (i.e. a simulation cell). This is required to be consistent with the minimum image convention typically used for systems with periodic boundary conditions.

However, for a crystal system possessing very small unit cells, the cut-off radius is often too small to properly account for the non-electrostatic potential energy interactions leading to inaccurate results. A solution to this problem would be to increase the size of the simulation cell beyond a unit cell. However, since the number of local energy minima scale drastically with system size, there will be a marked increase in the computation power required to locate the global minimum.

Podeszwa et al. (Phys. Chem. Chem. Phys., 2009, 11, 5512-5518) disclose the application of crystal structure prediction and molecular dynamics methods to the cyclotrimethylene trinitramine (RDX) crystal to explore the stability rankings of various polymorphs using a nonempirical potential energy function that describes the RDX dimer interactions and includes a Coulomb term for the electric potential energy. The energies of 500 high-density structures resulting from molecular packing were minimized and the 14 lowest-energy structures were subjected to isothermal-isostress molecular dynamics (NsT-MD) simulations. For the molecular dynamics simulation, the sizes were selected to ensure that the perpendicular widths between opposing surfaces of the simulation cell were at least twice the interaction potential cutoff distance (12 Å). In other words, the cutoff distance chosen was smaller than the dimensions of the simulation cell.

The present invention aims to at least ameliorate the aforementioned disadvantages by providing a method for predicting crystal structure of materials with unit cells of arbitrary size, without significantly increasing the computational cost.

**SUMMARY OF THE INVENTION**

According to a first aspect of the present invention, there is provided a method of determining one or more stable crystal structures, each crystal structure comprising a repeated unit cell, by calculating a configurational energy of the system with periodic boundary conditions having a plurality of particles, as set out in claim 1.

The present invention allows determination of the crystal structure for a system possessing unit cells of arbitrary size by removing the constraint of the simulation cell size being equal to or less than the unit cell of the crystal structure. The present invention overcomes this problem by using a supercell expansion method, allowing the use of unrestricted cut-off radii to accurately determine the total enthalpy of a system having a plurality of particles arranged in a crystal structure with any unit cell size. Accordingly, the present invention provides a more efficient method to locate the global minimum of the configuration energy, corresponding to a stable crystal structure.

Applications of such crystal structure determination include determination of crystal structure polymorphs for pharmaceutical applications (as noted above), minerology, crystallography and crystal engineering (to name but a few). As noted, by determining the (stable) crystal structure(s), experimental efforts can be guided.

The non-electrostatic pair potentials of particles include particles lying outside the supercell, but within the cut-off radius.

Furthermore, or alternatively, step iv) may consider additional image particles surrounding a particle using the standard minimum image convention to select said non-electrostatic pair potentials resulting from the interaction of the particle with closest particles or image particles located inside and/or outside the supercell. This ensures that (only) the closest image is taken into account when calculating the non-electrostatic pair potentials.

According to an embodiment of the present disclosure, the calculating step iv) may further comprise the steps of: calculating, for each particle located within a selected simulation cell, non-electrostatic pair potentials between the particle and any and all additional particles surrounding the particle within the cut-off radius.

Furthermore, the summing step v) may comprise the steps of: summing the distinct non-electrostatic pair potentials for each particle inside the selected simulation cell; and multiplying the summation by the number of simulation cells inside the supercell, to obtain configurational energy, and wherein the configurational energy is a non-electrostatic potential energy per supercell of the system.

Steps of the method may comprise calculating non-electrostatic pair potentials for each particle inside a selected simulation cell. The non-electrostatic pair potential for each particle inside the selected simulation cell may have contributions from any and all other particles located inside and outside the selected simulation cell within the cut-off radius if the cut-off radius extends beyond the selected simulation cell. The total potential energy of the simulation cell may then be obtained by summing all the distinct non-electrostatic pair potentials of particles inside the selected simulation cell. The total potential energy of the supercell, which comprises a plurality of simulation cells, may then be obtained by multiplying this sum by the total number of simulation cells. This provides a non-electrostatic configurational energy of the system, allowing the configurational energy to be determined using further methods (such as accounting for electrostatic interactions and the effect of external pressure).

The presently described method allows for a more accurate determination of a configurational energy of the system, without the expected increase the number of coordinates of the system. This reduces the required computation power for the subsequent optimisation process (such as Basin Hopping techniques) to find a global minimum of the system.

In an example, the total non-electrostatic pair potentials of a particle inside the selected simulation cell may also include or have contributions from particles lying outside the selected simulation cell, but which are still within the cut-off radius of the particle inside the supercell.

As noted above, it can be appreciated that the summation may be performed using standard minimum image convention to conserve the number of particles inside the supercell cell. This may ensure that the particle numbers inside the super cell are conserved - the particles positions are typically not fixed during the course of the simulation. Hence, there may be occasions when one or more particles may leave the supercell during the optimization method, causing jumps in the potential energy of the system. In this case, when the above summation is performed using standard minimum image convention, the number of particles inside the supercell is conserved.

The non-electrostatic potential energy per simulation cell (a traditional end result for crystal structure prediction used in conventional methods) can then be obtained by dividing the total non-electrostatic potential energy per supercell cell by the total number of simulation cells.

Configurational energy as described in the present disclosure accounts for both electrostatic and non-electrostatic potential energy, and may optionally further comprise contributions from a non-zero external pressure.

Where electrostatic interactions are considered, for example, where each particle comprises one or more multipoles, embodiments of the second aspect may comprise the steps of determining the electrostatic interactions between particles by determining contractions between particle multipoles that are then combined using an Ewald sum with the non-electrostatic interactions in the manner described above and below.

In examples, the step of converting the multipole interactions from a Cartesian coordinate system into a spherical harmonic may provide the multipole interactions in a form suitable for implementation into the Ewald sum.

It can be appreciated that the step of converting may comprise the step of: diagrammatically representing the multipole interactions as a series of nodes and spokes radiating from the nodes, wherein each node represents a symmetric multipole tensor defining a multipole of a particle and wherein the number of spokes radiating from each node is equal to the rank of the symmetric multipole tensor of that node. Additionally, the method may further comprise the step of conjoining spokes of interacting multipoles of particles to form spoked connections between the respective nodes, each spoked connection representing an interaction between the nodes. Furthermore, a degree of contraction acting between any two nodes may be equal to the number of spoked connections shared between two nodes.

This method provides a direct connection between the Cartesian and spherical harmonic representations, such that it becomes straightforward to transform between the two representations, and the final expressions lend themselves to being easily implemented in Ewald sum type methods.

The step of diagrammatic representing may further comprise the step of: for each node, braiding the spokes to transform each spoke of the node from Cartesian components into a spherical harmonic form. Additionally or alternatively, the step of diagrammatic representing may further comprise the step of: braiding the spoked connections between nodes on a piece by piece basis, wherein each piece constitutes a subset of spoked connections between the nodes.

In an embodiment, the symmetric multipole tensors may be traceless. This allows spherical harmonics to be used as an orthogonal basis to represent the symmetric multipole tensors.

A basin-hopping algorithm is generally a global optimisation approach which works by performing a walk on a transformed potential energy surface, where the transformed, or 'plateaued', potential energy surface is defined by the energy of the local minimum at each point, which is obtained by performing a numerical optimisation starting from the coordinates on that step. The transformed surface removes many of the barriers between minima, and it has been shown that walks on this surface have a greater chance of sampling low-lying minima, including the global minimum.

Accordingly a basin-hopping global optimisation algorithm may comprises the steps of: a) obtaining a local minimum $H(\mathbf{X})$ with coordinate $\mathbf{X}$ by employing a local optimisation algorithm; b) generating a random displacement vector $\Delta\mathbf{X}$ to obtain new trial coordinates $\mathbf{X^{trail}} = \mathbf{X} + \Delta\mathbf{X}$; c) accepting $\mathbf{X^{trail}}$ if $\mathbf{X^{trail}}$ produces separation between any two particles inside the simulation cell greater than or equal to a set minimum distance $r^{min}$, or rejecting $\mathbf{X^{trail}}$ rejecting if separation between any two particles inside the supercell is smaller than $r^{min}$; d) repeating steps b) to c) until an acceptable value of $\mathbf{X^{trail}}$ is found; e) calculating a transformed enthalpy $H^{min}(\mathbf{X^{trail}})$ per simulation cell by employing the local optimisation algorithm on $H(\mathbf{X^{trail}})$; f) accepting $H^{min}(\mathbf{X^{trail}})$ and setting $\mathbf{X^{trail}} = \mathbf{X}$ if the standard Metropolis Monte Carlo criterion is met: $R < \min[1, exp(-\beta(H^{min}(\mathbf{X^{trial}}) - H(\mathbf{X})))]$, where $R$ is a uniform random number between 0 and 1, and, where $k_B$ is Boltzmann's constant; otherwise, rejecting $H^{min}(\mathbf{X^{trail}})$ and returning $\mathbf{X}$ to the value of the last accepted local minimum before the rejection; and g) repeating steps b) and f) to calculate a plurality of local minima of $H(\mathbf{X})$. The global minimum of the system can then be the value of the local minimum that is the lowest from the plurality of calculated local minima of $H(\mathbf{X})$.

As noted above, in a next step a random displacement vector $\Delta\mathbf{X}$ may be generated to obtain new trial coordinates $\mathbf{X^{trail}} = \mathbf{X} + \Delta\mathbf{X}$, where $\mathbf{X}$ are the initial coordinates of the particles in the system. However, since basin-hopping approaches typically uses quite large Monte Carlo step sizes, the trial step could move a molecule unphysically close to another molecule, resulting in huge forces. This could cause problems for the local optimisation algorithm later on, making it hard to find the local minimum. To account for this, a number of possible of trial moves can be restricted such that the only configurations where every intermolecular particle pair is larger than a specified minimum distance $r_{min}$ are allowed. If a trial move does not satisfy this criterion, then a new trial move may be generated, until the algorithm finds a move which does. Accordingly $r^{min}$ may be constrained by a set maximum.

In some embodiments, the particles may be considered to be molecules. Accordingly $H(\mathbf{X})$ comprises contributions from intramolecular potential energy corresponding to a conformer of the molecule inside the simulation cell. Furthermore, in step g), calculating the plurality of local minima of $H(\mathbf{X})$ may comprise taking into account different conformers of the molecule from a conformer database, with each conformer corresponding to a local minimum of an intramolecular potential energy surface of the molecule. Additionally, step g) may further comprise calculating the plurality of local minima of $H(\mathbf{X})$ may further comprise calculating a transition probability, said transition probability indicating the probability of the molecule transitioning from one conformer to another conformer at a given temperature. Temperature may be varied to ensure that the system will eventually visit every conformer with a reasonable probability.

This embodiment uses a computational method, such as density functional theory (DFT), to generate an initial set of possible conformer structures, which are then used to determine a plurality of conformers having lowest local minima. The resulting best structures (i.e. those with lowest global minimum energies) can then be relaxed to produce new conformers that can in turn be fed back into the conformer database. Additionally or alternatively, conformers corresponding to local minima of the potential energy landscape may be identified as per the method above, to build an initial database of conformers. This initial set of conformers can then be used to determine possible crystal structures. The best structures (i.e. those with lowest energies) can then be relaxed using DFT to produce new conformers that can be augmented to the database of conformers in preparation for further crystal structure predictions. This can be repeated to improve the quality of the conformer database.

From the updated conformer database, likely crystal structures for a given conformer and their associated configurational energies can be determined. This allows more crystal structures to be predicted for these conformers until a 'true' global minimum for the system is determined. The total enthalpy may therefore be considered to include the intramolecular energies corresponding to the conformers of the molecule.

If the new trial coordinates are accepted, then a standard Metropolis Monte Carlo criterion may be applied to decide whether to accept or reject the transformed enthalpy with the new trial coordinates. Applying a standard Metropolis Monte Carlo criterion may increase the efficiency of the basin-hopping global optimisation algorithm by reducing the probability of encountering a large number of consecutive rejections.

If the standard Metropolis Monte Carlo criterion is not met, then **X** may be returned to the last accepted local minimum before rejection. If the criterion is met, however, then a new trial coordinate is generated and the process is repeated until a global minimum is found.

In one embodiment, the size of the supercell may be allowed to vary during the basin-hopping global optimisation procedure such that the supercell is always large enough to hold its cut-off radius.

In an embodiment the stable crystal structures are candidates for a pharmaceutical candidate, and wherein each crystal structure comprises a polymorph of the pharmaceutical candidate. This allows stable polymorphs to be determined for pharmaceutical candidate drugs, such that stable enantiomers or polymorphs of pharmaceutical products can be determined, reducing the time taken for clinical trials and allow for a more exhaustive search of the polymorph landscape than could necessarily be found by powder X-ray diffraction, in a fraction of the time (weeks vs months), and in a more automated fashion. In turn, this knowledge of polymorphs, and the most thermodynamically stable ones, can be used to inform/improve Powder -Xray Diffraction, and allow for testing to ensure that the most stable one is actually manufactured.

Additionally, regulatory bodies (e.g., the US FDA) demand that particular (biologically active) polymorphs are characterised properly in terms of their thermodynamic/kinetic stability. The present embodiment makes this less onerous than traditional crystal structure prediction techniques.

Whilst there are benefits for pharmaceutical product candidate searches and polymorph determination, the described methods may be used in other industries, such as materials discovery (or 'materials genomics'), e.g., photovoltaics, photoelectrochemical water-splitting, gas storage in caged crystal structures, etc. Ensuring thermodynamic stability of the final material produced is important in these industries to ensure that produced materials have stable crystal structures. Using the described techniques to determine the most stable candidate crystal structures, this could also be important for structural materials, e.g., quantifying the strength, etc.

Given a determined crystal structure, it is then possible to determine the potential properties of such a structure, and accordingly determine how to control the necessary parameters to promote such crystalline structure for a given chemical composition. Given that crystal structures can have such an important impact on the physiological response of a patient to pharmaceutical compounds, determination of crystal structure using the above method provides clear benefits in focussing experimental and research efforts.

The above described method provides a reliable technique for determining crystal structures without the need to undertake, potential harmful or dangerous crystal structure experiments.

It can be appreciated that for both the first, second and third aspects, the cut-off radius may be equal to or greater than a unit cell of the crystal structure.

According to a further aspect of the present invention, there is provided a computer program for executing the method according to any embodiment of the preceding aspects.

Accordingly, there may be provided a computer program, which when run on a computer, causes the computer to configure any apparatus, including a circuit, controller, sensor, filter, or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software implementation may be an assembly program.

For example, the computer program may be provided on a computer readable medium, which may be a physical computer readable medium, such as a disc or a memory device, or may be embodied as a transient signal. Such a transient signal may be a network download, including an internet download.

The aspects of the present disclosure allows for the crystal structure of complex solid state systems to be determined, for example pharmaceutical compounds, industrial crystalline materials and the like, such as metals, semiconductors, polymers, drugs in protein ligand complexes, etc.

It can be appreciated that the step of determining possible crystal structures may comprise the step of determining possible configurations of the crystal according to an analysis or an estimate of its chemical composition. Furthermore or alternatively, an iterative approach may be undertaken with relative configurations generated from and based on standard crystal structures.

Preferred embodiments of the present invention will now be described with reference to the drawings, of which:

**BRIEF DESCRIPTION OF DRAWINGS**

Figure 1 is schematic illustration of a known method for calculating a configurational energy of a system comprising a plurality of particles;

Figure 2 is schematic illustration of a method for calculating a configurational energy of a system comprising a plurality of particles according to the present invention;

Figures 3a - 3l show example crystal structures for water ice determined from the configurational energy calculated using the method of Figure 2.

It should be noted that the Figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments.

**DETAILED DESCRIPTION OF INVENTION**

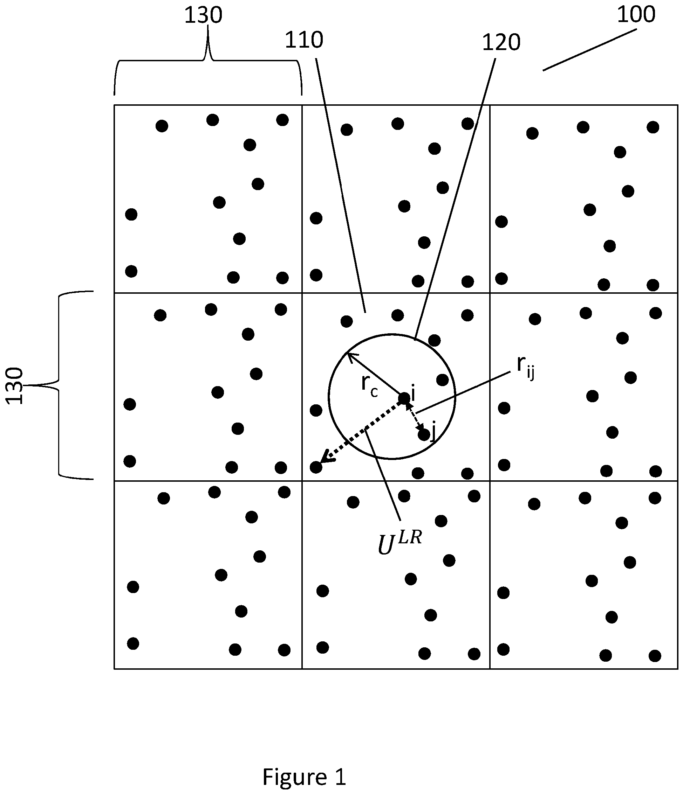

Figure 1 shows a known schematic technique for determining a configurational energy using a conventional method. In this method 100, unit cells 110 are defined from unit cell vectors 130 (shown in 2D in this figure - a third dimension would also be present). Each unit cell 110 defines a boundary inside which particles that make up the unit cell of the crystal structure are bound and is a repeated unit structure of the crystal.

In order to determine a configurational energy of the system 100, each particle inside a unit cell 110 is used to calculate a potential energy U between the selected particle and all other particles lying within a boundary 120 defined by a cut-off radius $r_c$. This includes particles lying outside of the boundary of the unit cell 110.

As an example, for a particle, pair potentials (i.e. the potential energy between two particles lying within a cut-off radius of each other) are calculated. So, for particle i, lying a distance $r_{ij}$ from a secondary particle j, a pair potential is calculated. A sum of these pair potentials, with account taken for long range potential energies $U^{LR}$ and contributions from external pressure allow a configurational energy to be calculated. Basin hopping or other global optimization techniques may then be used on the calculated configurational energy to converge to a global minimum that corresponds to a stable crystal structure.

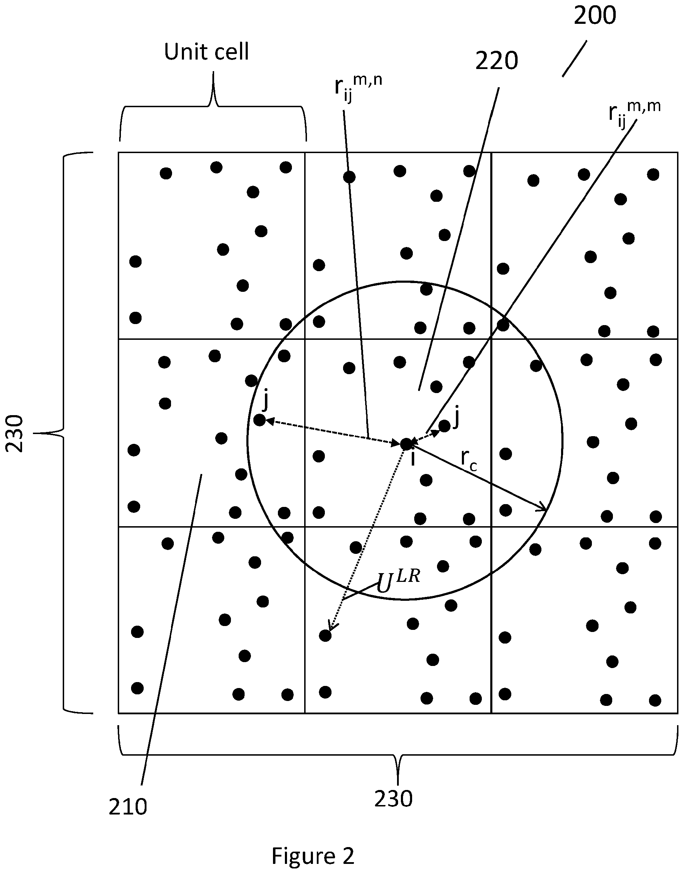

The method or technique used in the present invention is shown in Figure 2. In this example, a system 200 comprises a plurality of particles 212 arranged in a nominal crystal structure, for example, estimated based on the chemical composition of the system or by the first local minimum in the basin-hoping procedure encountered, before more minima are encountered methodically. In this example, unit or simulation cells 210 are chosen based on the estimated crystal structure having repeated crystal structure.

Supercell vectors 230 can then be defined based on the simulation cells such that an integer number of simulation or unit cells 210 fit into each supercell vector 230. Together the supercell vectors 230 (including a 3$^{rd}$ vector not shown) define a supercell of the crystal structure. This supercell encompasses an integer number of unit or simulation cells 210 of the system and so comprise a number of repeated crystal structure.

A similar technique to figure 1 can then be employed. However, it can be appreciated that the boundary 220 defined by a cut-off radius $r_c$ larger than the boundary in the known technique shown in figure 1. In particular, the larger cut-off radius includes particles lying well outside of the boundary of the unit cell 110. In fact, now consideration is given to the interaction of particles in the same position, but lying in adjacent unit or simulation cells 210.

As an example, for a particle, pair potentials (i.e. the potential energy between two particles lying within a cut-off radius of each other) are calculated. So, for particle i, lying a distance $r_{ij}^{m,m}$ from a secondary particle j1, a pair potential is calculated. However, the secondary particle j2, which lies in the same configurational position to particle j 1 in a different unit cell can also have the pair potential determined due to it lying a distance $r_{ij}^{m,n}$ away from particle i.

A sum of these pair potentials, with account taken for long range potential energies $U^{LR}$ and contributions from external pressure allow a configurational energy to be calculated. Basin hopping or other global optimization techniques may then be used on the calculated configurational energy to converge to a global minimum that corresponds to a stable crystal structure.

More particularly, for a system with periodic boundary conditions with $N$ particles per unit cell interacting under a non-electrostatic pair potential, where the energy of a pair of particles with separation r is given by $u(r)$, with $u(r) \rightarrow 0$ as $r \rightarrow \infty$; the configurational energy *per simulation cell U* is commonly taken to be approximated by:

$$U = \sum_{i}^{N} \sum_{\substack{j>i \\ \tilde{r}_{ji}<r_c}}^{N} u(\tilde{r}_{ji}) + U^{LR} \quad (1)$$

where $\tilde{r}_{ji} = |\tilde{r}_{ji}|$ , with $\tilde{r}_{ji}$ being the so-called minimum image of $\boldsymbol{r}_{ji} = \boldsymbol{r}_j - \boldsymbol{r}_i$, where $i$ and $j$ are the $i^{th}$ and $j^{th}$ particle inside one simulation cell. $U^{LR}(r_c)$ is a long-range correction to the energy, which approximates interactions outside of the cut-off sphere. Approximating the density of particles at $r > r_c$ by a uniform distribution $u(r)$, this long-range correction takes the form:

$$U^{LR} = 2\pi \frac{N^2}{V} \int_{r_c}^{\infty} u(r) r^2 dr \quad (2)$$

where $V = \boldsymbol{a} \cdot (\boldsymbol{b} \times \boldsymbol{c})$ is the cell volume, and, and are reciprocal cell vectors of a unit cell.

The condition $j > i$ for sum on the right hand side of equation 1 is to prevent the same pair-potentials from being included more than once. Therefore, equation 1 only sums distinct pair potentials inside the simulation cell. In addition, the sum in equation 1 is only over the pair potentials for which $\tilde{r}_{ji} < r_c$, where $r_c$ is the cut-off radius.

In addition, the total configurational energy per stimulation cell of the system also comprises the electrostatic interactions between particles. Details of how this can be estimated are explained below.

Also note, that in the case of a non-zero pressure, the above is to be supplemented by a $PV$ term, to give the *enthalpy* per simulation cell: $H = U + PV,$ where $P$ is the externally applied pressure and $V$ is the cell volume.

The minimum energy process is required to prevent jumps in the potential energy when one or more particles leave the simulation cell during the simulation. The minimum image and the cut-off radius $r_c$ work in tandem to restrict the energy sum to include only those translationally distinct pair interactions in the periodic system which lie inside the cut-off sphere. In standard approaches, the size of cut-off radius is restricted such that the associated cut-off sphere fits inside a unit cell. This is required to be consistent with the minimum image process, which always returns vectors inside one unit cell.

The maximum value of the cut-off radius $r_c$ then is determined by the largest sphere that can fit inside a triclinic cell, and it is not too difficult to show that, for a unit cell 110 of lattice vectors 130 $\boldsymbol{a}, \boldsymbol{b}, \boldsymbol{c},$ the radius of such a sphere must satisfy the fowling condition:

$$r_c \leq \frac{1}{2} min \left( \frac{1}{a^*}, \frac{1}{b^*}, \frac{1}{c^*} \right) \quad (3)$$

where $a^* = |\boldsymbol{a}^*|$ are magnitudes of the reciprocal cell vector, and similarly for $\boldsymbol{b}^*$ and $\boldsymbol{c}^*$.

For sufficiently fast converging interactions, the energy sum, including the long-range correction, is often quite a good approximation to the actual energy per unit cell, i.e. when the entire periodic system is taken into account, and, in the limit, it ought to converge to the ideal result. However, as the energy sum stands, the cut-off radius is always limited by the size of the simulation cell.

Thus, obtaining converged results requires that the simulation cell 210 be large enough to contain a sufficiently large cut-off sphere.

To overcome this problem, the present invention employs a supercell with cell vectors 230 $M_a\boldsymbol{a}, M_b\boldsymbol{b}, M_c\boldsymbol{c},$ where $\boldsymbol{a}, \boldsymbol{b}, \boldsymbol{c}$ are the cell vectors 130 of the original simulation cell. $M_a, M_b, M_c$ are integers, and where the supercell comprises M = $M_aM_bM_c$ replicas, each of which are identical to the original simulation cell.

The coordinates of the $i^{th}$ particle in the $m^{th}$ replica or unit/simulation cell 210 are given by : $r_i^m = r_i + R_m$ , where $\boldsymbol{R_m} = m_a\boldsymbol{a} + m_b\boldsymbol{b} + m_c\boldsymbol{c}$ is the lattice generated by integer multiples of the unit cell vectors 110.

Also, if $\boldsymbol{r}_{ji} = \boldsymbol{r}_j - \boldsymbol{r}_i$ is the pair vector between two particles in the simulation cell, then $r_{ji}^{n,m} = r_j^n - r_i^m = r_{ji} + R_{n-m}$ is the vector for the pair vector between particles across replicas.

A standard minimum image process can be applied to the supercell. Defining $\overline{r}$ to be the supercell minimum image of $r$, the supercell minimum image of $r_{ji}^{n,m}$ is given by:

$$\overline{r}_{ji}^{n,m} = r_{ji}^{n,m} - C^{super} \operatorname{nint}\left(C^{super-1} r_{ji}^{n,m}\right) \quad (4)$$

where

$$C^{super} = \begin{bmatrix} M_a a_x & M_b b_x & M_c c_x \\ 0 & M_b b_y & M_c c_y \\ 0 & 0 & M_c c_z \end{bmatrix} \quad (5)$$

is the matrix of supercell cell-vectors (here presented in upper-triangular form).

The cut-off radius $r_c$ now has to fit inside the supercell, and so must satisfy the following condition (c.f. equation 3):

$$r_c \le \frac{1}{2} min \left(\frac{M_a}{a^*}, \frac{M_b}{b^*}, \frac{M_c}{c^*}\right) \quad (6)$$

Suppose that the simulation cell vectors 120 are allowed to change during the course of a simulation. The cut-off radius $r_c$, as usual, is held fixed during the course of the simulation, but the size of the supercell is *not* fixed, and the $M_a$, $M_b$, $M_c$ integers controlling its dimensions can be adjusted on the fly such that the supercell is always just large enough to hold its cut-off sphere. These values can be calculated by inverting equation 6, to obtain:

$$M_a = \operatorname{int}(2r_c a^*) + 1, \quad (7)$$

and similarly for $M_b$ and $M_c$, where the int($x$) function (for positive arguments) rounds down to the nearest integer.

The energy per replica (i.e. per simulation cell), is given by summing over every pair interaction in the supercell smaller than the cut-off radius, and dividing by the number of replicas, $M$. Excepting the long-range correction, this energy is given by:

$$U = \frac{1}{2}\frac{1}{M}\sum_i^N \sum_j^N \sum_m^M \sum_{m'}^M u(\overline{r}_{ji}^{m',m}) \quad (8)$$

where the sums over $m$ and $m'$ are over all $M$ replicas in the supercell, and where it is assumed that the sum excludes the non-physical $\tilde{r}_{ii}^{m,m} = 0$ terms describing the interaction of each particle with itself. As usual, only interactions inside the cut-off radius are counted.

In principle, the above approach should work, but it is very wasteful, as it involves counting translationally equivalent pair interactions multiple times. In fact, it can be shown that equation 8 sums over every distinct $ij$ interaction $M$ times, once for each replica in the supercell.

To remove the over-counting, first consider the following identity

$$\sum_m^M u(\overline{r}_{ji}^{m,k}) = \sum_m^M u(\overline{r}_{ji}^{m,l}) \quad (9)$$

which holds because the LHS sums the interaction of $r_i^k$ with every replica of $r_j$ in the supercell, and the RHS sums the interaction of $r_i^l$ with every replica of $r_j$ in the supercell. However, in periodic boundary conditions, $r_i^k$ and $r_i^l$ describe the same particle, just in different replicas, so the two sums must be equal.

Using the above result, it follows that:

$$\sum_{\underset{m m'}{M M}} u(\overline{r}_{ji}^{m',m}) = \sum_{\underset{m m'}{M M}} u(\overline{r}_{ji}^{m',0}) = M \sum_{\underset{m}{M}} u(\overline{r}_{ji}^{m,0}) \quad (10)$$

which, when applied to equation 8, gives the energy sum as a single sum over replicas:

$$U = \frac{1}{2} \sum_{i}^{N} \sum_{j}^{N} \sum_{m}^{M} u(\overline{r}_{ji}^{m,0}) \quad (11)$$

There still exists an over-counting problem because both $ij$ and $ji$ are summed over. As for equation 1, this can be avoided by restricting the sum to $j > i$. This works fine for the $i \neq j$ interactions, but the $i = j$ interactions cannot be summed this way. Considering these interactions separately, and using the identity $r_{ii}^{m,0} = \boldsymbol{R}_m$ , then the final form for the supercell version of the energy sum is:

$$U = \sum_{i}^{N} \sum_{j>i}^{N} \sum_{m}^{M} u(\overline{r}_{ji}^{m,0}) + \frac{N}{2} \sum_{m \neq 0}^{M} u(\overline{R}_m) \quad (12)$$

It can be seen from equation 12 that the cut-off radius is no longer restricted to fit inside the simulation cell, but which can now be made arbitrarily large. And as usual, it is possible to supplement the above with the long-range correction of equation 2, to take into account interactions outside the cut-off sphere.

Figure 2 illustrates the above supercell method in real space. The process is very similar to the standard energy-sum of equation 1, except that it involves a sum over replicas; the minimum images are now applied with respect to the supercell cell-vectors; and the interactions of particles with their replicas now also have to be summed.

Now that a method for handling small unit cells has been found, the global minimum of the system can be found using a variant of the basin-hopping method.

In the basin-hopping method, a simulation proceeds on a transformed potential energy surface U', which is given by:

$$U'(\boldsymbol{X}) = U^{min}(\boldsymbol{X}) \quad (13)$$

where $\boldsymbol{X} = X_1, X_2, ... X_N$ are $N$ coordinates specifying the system, and $U^{min}(\boldsymbol{X})$ is the local minimum, starting from $\boldsymbol{X}$, under the potential energy surface $U$. That is, $U'(\boldsymbol{X})$ is obtained by using a local-optimisation algorithm, which starts from initial coordinates $\boldsymbol{X}$, and then travels downhill until it finds a local minimum. Also note that in the case of an externally applied pressure, a transformed enthalpy surface, $H'(\boldsymbol{X}) = H^{min}(\boldsymbol{X})$, will be used.

In this method, a Metropolis Monte Carlo algorithm is commonly used to walk around the transformed potential energy surface. This will result in the system visiting a succession of different local minima, with the hope that, if the simulation is run long enough, that it may eventually find its way to the global minimum.

Most local-optimisation algorithms require both the energy and the derivatives of the energy with respect to each coordinate i.e. they need to be supplied with a subroutine which returns the $N$ derivatives for any value of $\boldsymbol{X}$. To make it computationally more efficient, these derivatives usually need to be implemented analytically.

In the present case, a convenient set of coordinates is given by: i) The $3N_{mol}$ molecular centre-of-mass displacements $f_{ia}^{com}, f_{ib}^{com}, f_{ic}^{com}$ , for $i = 1.. N_{mol}$, where $N_{mol}$ is the number of molecules per unit cell (with the 3 accounting for x, y and z), ii) The $3N_{mol}$ Euler angles $\phi_i, \theta_i, \psi_i$ specifying the rotation of each molecule about its centre of mass, and iii) the 6 independent lattice parameters, $a_x, b_x, b_y, c_x, c_y, c_z$. Thus, the code needs to be furnished with analytic derivatives:, and (and like components).

A problem encountered in using the Monte Carlo algorithm on the transformed surface is that often there would be a large number of consecutive rejections; with the Monte Carlo walk effectively getting stuck in regions where it is surrounded by multiple higher minima.

To overcome this, a modified basin-hopping algorithm (basin-escape algorithm) is used in which the walk is started at a local minimum and takes steps until it encounters a rejection, in which case, the walk is reset to the position of its local minimum, $\boldsymbol{X} \rightarrow \boldsymbol{X}^{min}(\boldsymbol{X})$, from where it takes the next step on the next iteration of the walk. It is often the case that this local minimum is the same as the one it started from, but it need not be, as in some cases the walk will have successfully escaped the basin of one minimum to another. In the latter case a rejection will result in walk being reset to the coordinates of the new minimum.

It was found that this simple zero-parameter modification substantially improves the efficiency of the algorithm, as it essentially solves the problem of the walk getting stuck in high-rejection regions.

Basin-hopping approaches typically use quite large Monte Carlo step sizes, and another problem encountered was that occasionally the trial step can move a molecule unphysically close to another molecule, resulting in very large force. This could cause problems for the local optimisation algorithm, making it hard to find the local minimum.

To solve this problem, the number of possible trial moves that the Monte Carlo walk was allowed to take was restricted, such that valid trial moves are configurations where every intermolecular particle pair is larger than a specified minimum distance. If a trial move does not satisfy this criterion, then a new trial move is generated until the algorithm finds a move which satisfies this criterion.

The $\Delta X_i$ random displacements required to produce new trial coordinates are chosen from, for example, Gaussian distributions. However, it may be appreciated that other uniform distribution can also be used. More explicitly, the displacements in each fractional coordinate are taken from a Gaussian distribution , with sigma values $\sigma_f$, and a random variable $X_f$, the displacements in the Euler angles are taken from a Gaussian distribution with sigma values $\sigma_e$, and the displacements in each of the six cell vector components are taken from a Gaussian distribution with sigma values $\sigma_c$. The values of $\sigma_f$, $\sigma_e$, $\sigma_c$ are adjustable parameters, which affect the efficiency of the basin-hopping. The inverse temperature, $\beta = \frac{1}{k_b T}$, where $k_B$ is Boltzmann's constant and $T$ is temperature, is also an adjustable parameter, and should generally be chosen such that the system has a reasonable probability of sometimes accepting a move to higher energy/enthalpy, so that it can overcome barriers. However, the temperature should not be made so large that the walk spends essentially all of its time exploring high-energy configurations.

Point charge electrostatic models are typically usually used to calculate the electrostatic potential energy of particles, where an Ewald summation is generally performed to ensure rapid convergence of the energy sum. In recent years, several methods have been developed to include higher order terms in the multiple expansion of the charge distribution (where the terms in the next three ranks are referred to by names quadrupole, octopole and hexadecapole etc.). The challenge then is to identify how to modify the Ewald summation, or similar algorithm, such that it can properly handle the higher order multipoles interactions.

An example derivation of the multipole interaction in spherical harmonics which does not require any detailed technical knowledge of the theory of spherical harmonics is detailed below.

Essentially, the method involves deriving the multipole interactions in terms of Cartesians. This interaction is then converted into a spherical harmonic form. This is done with the aid of three key ideas: i) the use of a diagrammatic representation of the interaction between multipole sites, which greatly clarifies the math - as it turns out the complete interaction can be calculated in a 'sum over diagrams' type sense; ii) the use of spherical harmonics to construct an orthogonal basis for the traceless multipole tensors, such that the Cartesian to spherical harmonic conversion can be achieved by way of Stone's tables of spherical harmonics; iii) the transformation of the Cartesians into spherical harmonics on a piece by piece basis, where each piece constitutes a *subset* of the full number of that multipole's Cartesian components, in a process referred to as *braiding;* a diagrammatic metaphor for how the transformation intertwines Cartesians through taking linear combinations. Below is a summary of this method.

Consider a cluster of $N_c$ charges, with charges $q_m$, at positions $r + \Delta r_m$, where the $\Delta$ is used to signify that the displacements are typically quite small (at least, they are closer to **r** than any test sites probing the fields from the distribution). We then define the rank-n cartesian multipole tensors, $\textbf{\textit{M}}^{(n)}$, of the distribution, with respect to **r**, according to

$$\textbf{\textit{M}}^{(n)} = \frac{1}{n!} \sum_m^{N_c} q_m \Delta \textbf{\textit{R}}^{m(n)} \quad (14)$$

where $\Delta \textbf{\textit{R}}^{m(n)} = \Delta \textbf{\textit{r}}^m \Delta \textbf{\textit{r}}^m \Delta \textbf{\textit{r}}^m ..$ is a tensor product, with cartesian components $\Delta R^{m(n)}_{\alpha\beta\gamma..} = \Delta r^m_\alpha \Delta r^m_\beta \Delta r^m_\gamma ..$ , in which each greek index is one of *x, y, z,* and there being n factors of type $\Delta r^m_\alpha$ in the product.

Note that in equation 14 and for the rest of the description, the inverse factorial is subsumed in the definition of each multipole, but other methods may use different conventions.

The rank 0 multipole, $M^{(0)}$, which is a scalar, is just the sum of the charge. The rank 1 multipole $\textbf{\textit{M}}^{(1)}$ multipole is referred to as the *dipole* moment, and is a vector with components $M^{(1)}_x$, $M^{(1)}_y$, $M^{(1)}_z$ . The rank 2 multipole, $\textbf{\textit{M}}^{(2)}$ is

called the *quadrupole,* and has 9 components: $M_{xx}^{(1)}, M_{xy}^{(1)}, M_{xz}^{(1)}$ , etc., and the rank 3 multipole with 27 components is called the *hexadecapole,* and so on

It is clear from their definition in equation 14 that each multipole tensor is symmetric under any permutation of its indices. For example, for the rank-3 multipole tensor, $M_{\alpha\beta\gamma} = M_{\alpha\gamma\beta} = M_{\beta\gamma\alpha} = M_{\beta\alpha\gamma} = M_{\gamma\alpha\beta} = M_{\gamma\beta\alpha}.$ In addition, it can be shown that the same is true in any axis frame. This permutation symmetry means that the multipole tensors belong to a class that are referred to as *symmetric* tensors.

The multipoles are useful because the electrostatic properties of the cluster can often be written in terms of a rapidly converging series over multipole moments of increasing rank, rather than having to sum over the individual charges themselves. To see this, place the charge distribution in a background non-uniform electrostatic potential, $\phi(\boldsymbol{r})$. Then, the electrostatic energy, $U(\boldsymbol{r}),$ of the cluster due to $\phi(\boldsymbol{r})$ is given by:

$$U(\boldsymbol{r}) = \sum_m q_m \phi(\boldsymbol{r} + \Delta\boldsymbol{r}_m) \quad (15)$$

Now, using the inner product notation $\langle \boldsymbol{A}^{(n)}, \boldsymbol{B}^{(n)} \rangle$, where $\langle \boldsymbol{A}^{(n)}, \boldsymbol{B}^{(n)} \rangle$ indicates an inner product over tensors $\boldsymbol{A}^{(n)}$ and $\boldsymbol{B}^{(n)}$, it can be shown that:

$$\langle \boldsymbol{A}^{(n)}, \boldsymbol{B}^{(n)} \rangle = \sum_{\alpha\beta\gamma..} A_{\alpha\beta\gamma..}^{(n)} B_{\alpha\beta\gamma..}^{(n)} \quad (16)$$

The Taylor series expansion of $\phi(\boldsymbol{r} + \Delta\boldsymbol{r}_m)$ can be written as:

$$\phi(\boldsymbol{r} + \Delta\boldsymbol{r}) = \sum_{n=0}^{\infty} \langle \Delta\boldsymbol{R}^{(n)}, \nabla^{(n)} \rangle \rangle \phi(\boldsymbol{r}) \quad (17)$$

where $\nabla^{(n)} = \nabla\nabla\nabla..$with components ...

A more concise way of writing the above is as:

$$U(\boldsymbol{r}) = \sum_{n=0}^{\infty} \langle \boldsymbol{M}^{(n)}, \phi^{(n)}(\boldsymbol{r}) \rangle \quad (18)$$

where $\phi^{(n)}(\boldsymbol{r})$ are rank n field tensors, created from repeated directional derivatives of $\phi(\boldsymbol{r})$, according to:

$$\phi^{(n)}(\boldsymbol{r}) = \nabla^{(n)}\{\phi(\boldsymbol{r})\} \quad (19)$$

Similarly, it can be shown from the elementary theory of multipoles, and from using another Taylor series expansion, that the electrostatic potential at r, from a charge cluster around the origin, with multipoles $\boldsymbol{M}^{(n)}$ is given by:

$$\phi(\boldsymbol{r}) = \sum_{n=0}^{\infty} (-1)^n \langle \boldsymbol{M}^{(n)}, \nabla^{(n)} \rangle \left\{ \frac{1}{r} \right\} \quad (20)$$

where $4\pi\varepsilon_0 = 1$ to simplify the formulae.

Thus, assuming the above strongly converges, to calculate the electrostatic potential from the charge cluster it is justified to ignore the fine details of the charge distribution and just working with its multipoles up to a maximum rank, where these multipoles are calculated from the multipole expansion of the charge distribution in equation 14, and where the multipoles are placed on the multipole expansion site, in this case at the origin.

Now consider the traces of the multipole moments. In the rank-2 case there is just one trace, which is given by $Tr[\boldsymbol{M}^{(2)}] = \sum_{\alpha} M_{\alpha\alpha}^{(2)}$ , and it will prove useful to define the traceless rank 2 tensor $M_{\alpha\beta}^{0}$ from:

$$M_{\alpha\beta}^0 = M_{\alpha\beta} - \delta_{\alpha\beta} Tr[\boldsymbol{M}^{(2)}]/3 \quad (21)$$

where $Tr[\boldsymbol{M}^{0(2)}] = 0$ (by design).

In terms of traceless and trace components, the energy of the rank-2 multipole tensor in a background electrostatic potential is given by:

$$\sum_{\alpha\beta} M_{\alpha\beta} \phi_{\alpha\beta}(\boldsymbol{r}) = \sum_{\alpha\beta} M_{\alpha\beta}^0 \phi_{\alpha\beta}(\boldsymbol{r}) + \frac{Tr[\boldsymbol{M}^{(2)}]}{3} \sum_{\alpha} \phi_{\alpha\alpha}(\boldsymbol{r}) \quad (22)$$

The last term in the above involves the Laplacian of the electrostatic potential. However, from elementary electrostatics it can be shown that the Laplacian vanishes in free space, and so the last term is zero. Thus, the trace of the quadrupole makes no contribution to the total energy and the traceless quadrupole can always be used in its place. Similarly, the traces of the higher rank multipoles can be removed, as they do not contribute to the energy.

It may be argued that in real systems the Laplacian is not zero, because any real atomic site will experience a non-zero charge-density from the inter-atomic and inter-molecular charge-clouds from the other particles. However, the standard multipole expansion for the interatomic electrostatic energy does not account for such effects, and they will henceforth be assumed to be zero.

It is often also more convenient to work with traceless form of the $\boldsymbol{R}$ tensors. From a known theorem it holds that the traceless forms of the $\boldsymbol{R}$ tensors may be defined from the repeated gradients of $1/r$ via:

$$\boldsymbol{S}^{(n)} = (-1)^n \frac{r^{2n+1}}{(2n-1)!!} \nabla^{(n)} \left\{\frac{1}{r}\right\} \quad (23)$$

where $\boldsymbol{S}^{(n)}$ is the symbol used for the traceless $\boldsymbol{R}^{(n)}$ tensor, and where the first few terms are given by: $S^0 = 1$, $S_\alpha^{(1)} = r_a$, , and where the tracelessness of $\boldsymbol{S}^{(n)}$ follows from the vanishing laplacian of $1/r$.

It is possible to refer to $\boldsymbol{S}^{(n)}$ as the *Maxwell Cartesian spherical harmonics,* given that these gradients were investigated by James Clerk Maxwell, and given that, although they are not orthogonal, they behave in many senses like the Cartesian analogue of the spherical harmonic polynomials, of which more later.

Substitution of equation 23 the above into equation 20 gives:

$$\phi(\boldsymbol{r}) = \sum_{n=0}^{\infty} \frac{(2n-1)!!}{r^{2n+1}} \langle \boldsymbol{M}^{(n)}, \boldsymbol{S}^{(n)} \rangle = \sum_{n=0}^{\infty} \frac{(2n-1)!!}{r^{2n+1}} \langle \boldsymbol{M}^{(n)}, \boldsymbol{R}^{(n)} \rangle \quad (24)$$

where the last term above comes from recognising that since $\boldsymbol{M}^{(n)}$ are traceless, then the traces of $\boldsymbol{R}^{(n)}$ make no contribution to the $\langle \boldsymbol{M}^{(n)}, \boldsymbol{R}^{(n)} \rangle$ inner product (using the same argument used for the vanishing contribution of the multipole traces in the $\langle \boldsymbol{M}^{(n)}, \nabla^{(n)} \rangle \{1/r\}$ inner product). Thus, given that $\boldsymbol{S}^{(n)}$ are the traceless form of $\boldsymbol{R}^{(n)}$, we have that $\langle \boldsymbol{M}^{(n)}, \boldsymbol{R}^{(n)} \rangle$ and $\langle \boldsymbol{M}^{(n)}, \boldsymbol{S}^{(n)} \rangle$ are identical.

The gradients play a central role in the theory of multipoles, as should already be evident from the last section. However, the Ewald sum analogue of the multipole interaction requires the calculation of more general gradients, in which spherically symmetric functions, here written as $B(r)$, are substituted for the $1/r$ terms in expressions like equation 20. That is, instead of calculating the gradients, it is now more desirable to find gradients $\nabla^{(n)}\{B_0(r)\}$, with the knowledge that if the results for the non-Ewald regular multipole expansion is required then can simply be set in the final expressions.

In particular, the Ewald sum analogue to the normal multipole formulae requires using the kernel, where erfc(x) is the complimentary error function, which corresponds to the interaction of a unit charge with a negative Gaussian 'screening' density, where the screening density is of the form $\rho(r) = -Aexp(-a^2 r^2)$ (suitably normalised).

To simplify these gradient calculations radial functions, $B_m(r)$, can be defined, where the zeroth term is given by $B_0(r) = B(r)$, and with the higher order terms defined according to:

$$B_{m+1}(r) = -B_m'(r)/r \quad (25)$$

from which it follows that if , then, , and, in general, , where !! is the double factorial.

As might be expected, the expressions are somewhat more complicated when the kernel is used, but it has been shown how the higher order functions for this case can be generated by way of a simple recursive procedure.

It is instructive to calculate the first few terms in the directional gradients of the $B_0(r)$ functions. Begin by recalling that for a general spherically symmetric function, $f(r)$, it can be shown that. It then follows that:

$$\frac{\partial}{\partial r_\alpha} B_m(r) = -r_\alpha B_{m+1}(r) \quad (26)$$

from which the first two directional derivatives evaluate to:

$$\frac{\partial B_0(r)}{\partial r_\alpha} = -B_1(r) r_\alpha \left(= -\frac{r_\alpha}{r^3}\right) \quad (27)$$

and

$$\frac{\partial^2 B_0(r)}{\partial r_\alpha r_\beta} = B_2(r) r_\alpha r_\beta - B_1(r) \delta_{\alpha,\beta} \left(= \frac{1}{r^5}\left(3 r_\alpha r_\beta - \delta_{\alpha,\beta} r^2\right)\right) \quad (28)$$

where the terms in brackets are the results for the particular choice of , and where these terms can be written in terms of the Maxwell Cartesian spherical harmonics encountered in equation 23.

It is of course possible to continue calculating higher order terms in this fashion, but the main point to make here is that the first two directional derivatives of $B_0(r)$ above contain terms in $B_l(r)$, for some value of $l$, and, in general, by induction it can be shown that gradient terms in every order can be expressed as a series in $B_l(r)$.

Returning to the multipole problem, suppose that there are two multipole sites, $i$ and $j$, at locations $r^i$ and $r^j$ respectively, each of which carry a set of multipoles of different ranks, where the multipoles are placed at the site locations. Then the electrostatic energy of this pair is due to each multipole on site $i$ interacting with every multipole on site $j$, which can be determined by first evaluating the field at $r^j$ from the multipoles at $r^i$ according to equation 20, and then calculating the energy of the multipoles on j in the presence of that field according to equation 18. Labelling this energy $U^{ji}(r^{ji})$, it gives:

$$U^{ji}(\boldsymbol{r}^{ji}) = \sum_{d_j=0}^{\infty} \langle \boldsymbol{M}^{j(d_j)}, \nabla_j^{(d_j)} \rangle \sum_{d_i=0}^{\infty} (-1)^{d_i} \langle \boldsymbol{M}^{i(d_i)}, \nabla_j^{(d_i)} \rangle \{ B_0(r^{ji}) \} \quad (29)$$

where $r^{ji} = r^j - r^i$ is the inter-site vector, $r^{ji} = |r^{ji}|$, $\nabla_j^{(d_i)}$ are the gradients with respect to $r^j$ and where the more general form containing the $B_0(r^{ji})$ kernel is used.

Similarly to how it was shown that every order term in the repeated gradients of $B_0(r)$ contain a series in $B_l(r)$, it is not difficult to see that the same must be true for the interaction energy, $U^{ji}(r^{ji})$, and, this can be made clear by collecting terms in $B_l(r^{ji})$ to write:

$$U^{ji}(\boldsymbol{r}^{ji}) = \sum_{l=0}^{\infty} B_l(r^{ji}) G_{ji}^l(\boldsymbol{r}^{ji}) \quad (30)$$

where the $G_{ji}^l(\boldsymbol{r}^{ji})$ functions can be thought of as coefficients in $B_l(r^{ji})$.

Evaluating these functions would seem to require calculating and summing over the $\nabla^{(n)}\{B(r)\}$ terms 'by hand', as it were, which would involve much tedious algebra. However, a closed form solution is known, and is given by:

$$G_{ji}^l(\boldsymbol{r}^{ji}) = \sum_{d_i+d_c+d_j=l} C_{d_i,d_c,d_j} \langle (\boldsymbol{M}^{i(d_i+d_c)}.d_i.\boldsymbol{R}^{ji(d_i)}), (\boldsymbol{M}^{j(d_j+d_c)}.d_j.\boldsymbol{R}^{ji(d_j)}) \rangle \quad (31)$$

where $\boldsymbol{R}^{(n)} = \boldsymbol{rrr}...$; the multipoles are all assumed to be traceless; the notation $\boldsymbol{A}.d.\boldsymbol{B}$ indicates a d-fold contraction over the tensor indices of $\boldsymbol{A}$ and $\boldsymbol{B}$; $d_i$ is the number of contractions in the bracket containing $\boldsymbol{M}^i$; $d_j$ is the number of contractions in the bracket containing $M^j$; $d_c$ is the number of contractions acting in the centre, between the two brackets, which are expressed as an inner product; and where $C_{d_i,d_c,d_j}$ are integer combinatorial coefficients, given by:

$$C_{d_i,d_c,d_j} = (-1)^{d_j} \frac{(d_c+d_i)!(d_c+d_j)!}{d_i! d_c! d_j!} \quad (32)$$

The sum in equation 31 is over all $d_i$, $d_j$, $d_c$, where $d_i + d_c + d_j = l$; $d_i$, $d_c$, $d_j \geq 0$, i.e. the sum is over all possible terms having $l$ contractions.

Equation 31 will be referred to as the 'multipole interaction generating formula', as it generates all the terms in the multipole-multipole expansion of the electrostatic energy.

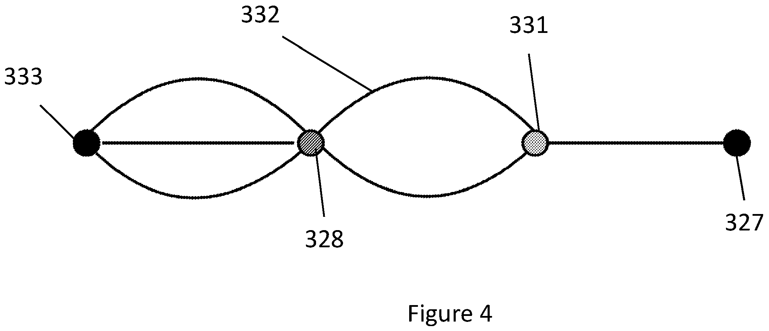

It is possible to construct a diagrammatic representation of the multipole interaction generating formula of equation 31, where one such diagram is shown in figure 4. It shows one term in the multipole interaction generating formula for I = 6, corresponding to $(M^{i(5)} : R^{ji(3)}) : (M^{i(3)}.R^{ji(1)})$, where the nodes from left to right represent $R^{ji(3)}$ (circle 333), $M^{i(5)}$ (circle 328), $M^{i(3)}$ (circle 330) and $R^{ji(1)}$ (circle 327). The rules being that each node represents a different tensor, where its rank is given by the number of spokes 332 radiating from the node in question; the number of spokes 332 shared between two nodes (i.e. the number of connected spokes) is equal to the degree of the contraction acting between the corresponding tensors; and the sign of the diagram is taken to be odd when there are an odd number of bonds connecting the $j$ multipole with its $R$ tensor.

Also note that no tensor or node 326, 328, 330, 327 may bond to itself. Given that a self-bond corresponds to taking the partial trace of a tensor, this rule follows directly from the fact that the traces of the $M$ and $R$ tensors make no contribution to the multipole interaction generating formula.

Generally, in addition to the total energy, forces, multipole fields, angular derivatives and torques are also required when implementing multipole interactions into molecular simulation code.

Let $F^{ji}(r_{ji}) = -F^{ij}(r_{ji})$ be the force on multipole site $j$ due to its interaction with multipoles site $i$, where the total

$$F^j = \sum_{i}^{N} F^{ji}(r^{ji})$$

force on $j$ is given by .

The force is found through taking the gradient of equation 30 and evaluates to:

$$F^{ji}(r^{ji}) = -\frac{\partial}{\partial r^j} U^{ji}(r^{ji}) = r^{ji} \sum_{l=0}^{\infty} B_{l+1}(r^{ji}) G_{ji}^l(r^{ji}) - \sum_{l=0}^{\infty} B_l(r^{ji}) \frac{\partial}{\partial r^j} G_{ji}^l(r^{ji}) \quad (33)$$

where equation 27 was used to find the gradient of the $B_l(r^{ji})$ function, and where the functions are found from taking the gradient of equation 32, and are given by:

$$\frac{\partial}{\partial r^j} G_{ji}^l(r^{ji}) = \sum_{d_i+d_c+d_j=l} C_{d_i,d_c,d_j} [d_i(M^{i(d_i+d_c)}.d_i - 1.R^{ji(d_i-1)}).d_c.(M^{j(d_j+d_c)}.d_j.R^{ji(d_j)})$$
$$+ d_j(M^{i(d_i+d_c)}.d_i.R^{ji(d_i)}).d_c.(M^{j(d_j+d_c)}.d_j - 1.R^{ji(d_j-1)})] \quad (34)$$

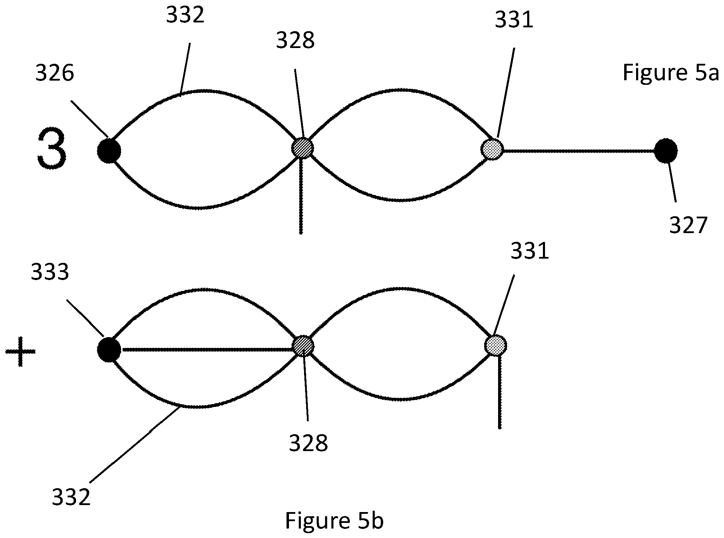

A diagrammatic representation of one force term is shown in figures 5a and 5b, which correspond to taking the gradient of the interaction from figure 4, and equates to $3(M^{i(5)}(328): R^{ji(2)}(326)): (M^{j(3)}(331).R^{ji(1)}(327)) + (M^{i(5)}(328) : R^{ji(3)}(333)): M^{j(3)}(331)$.

Comparing figures 4 and 5a, 5b, it can be seen that taking the gradient results in breaking one of the bonds connecting either the i or j multipole with its $R$ tensor. This leaves the multipole with a bare (or unbonded) spoke, which when taken as a whole gives a diagrammatic representation of a rank 1 vector.

Although an expression for the total energy of the system is already provided, an arguably more elegant expression for the energy is in terms of multipole field tensors can be written as:

$$U = \frac{1}{2} \sum_{i}^{N} \sum_{n=0}^{\infty} \langle M^{i(n)}, \phi^{i(n)} \rangle \quad (35)$$

where $\phi^{i(n)}$ is the rank n field tensor on site i, which is defined from:

$$\phi^{i(n)} = \frac{\partial U}{\partial M^{i(n)}} \quad (36)$$

Equation 35, which is written in terms of multipole fields, has the distinct advantage that it allows for an automatic decomposition of the total energy into contributions from the different multipole ranks, i.e. charge, dipole, quadrupole etc. And another reason to calculate the fields is that it greatly simplifies calculation of angular derivatives and torques, to be given in the next section.

The fields can be calculated by finding the derivative of the energy with respect to each multipole. Looking at just one pair of multipole sites, the field on multipole site j due to the multipoles on site i is given by

$$\phi^{ji(n)} = \frac{\partial U^{ji}(r^{ji})}{\partial M^{j(n)}} = \sum_{l=0}^{\infty} B_l(r^{ji}) \frac{\partial}{\partial M^{j(n)}} G_{ji}^l(r^{ji}) \quad (37)$$

where, taking the derivative of equation 31 with respect to the multipoles we obtain

$$\frac{\partial}{\partial M^{j(n)}} G_{ji}^l(r^{ji}) = SYMM\left[ \sum_{\substack{d_i+d_c+d_j=l \\ d_c+d_j=n}} C_{d_i,d_c,d_j}\left( M^{i(d_i+d_c)}. d_i. R^{ji(d_i)} \right) R^{ji(d_j)} \right] \quad (38)$$

where SYMM indicates that the resulting tensor elements are to be symmetrised over all index permutations, and for their traces to be removed.

This final step is necessary to ensure that the field tensors have the same symmetries as their corresponding multipoles, given that per their definition, the field tensors must be unchanged with respect to any permutation of their indices. And the removal of the traces is because the field traces make no contribution to the energy, and so it makes sense that these are set to zero.

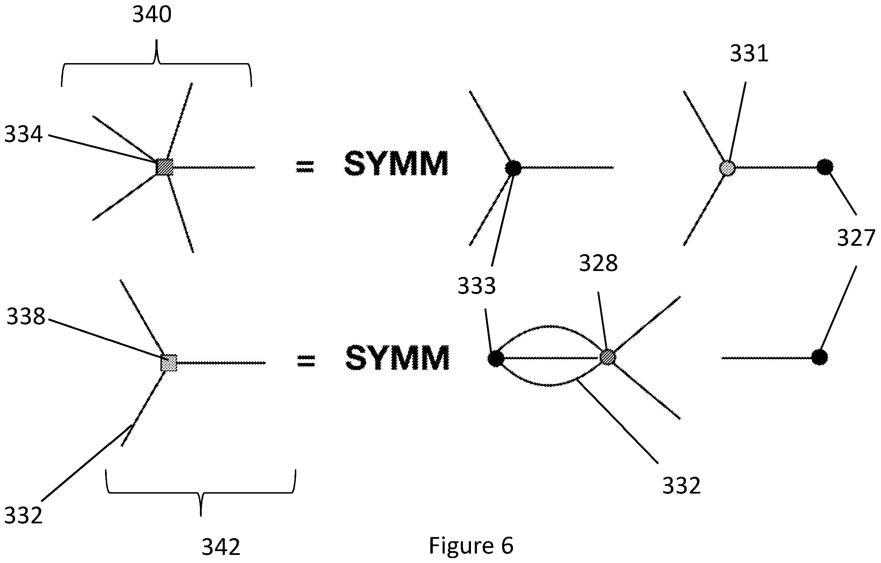

A diagrammatic representation of the field calculation is shown in figure 6, in which a rank 5 field (334) on tensor $M^{i(5)}$(328), and a rank 3 field on tensor $M^{j(3)}$ (331), corresponding to the multipole interaction in figure 4 or figure 5, are shown. The spokes 332 radiating from field tensor nodes (334, 338) represent multipole field tensors, of rank given by their number of spokes 332. The star 340 with the central node 334 represents the rank-5 field tensor on the ith site, and the star 342 with the central node 338 represents the rank-3 field tensor on the jth site. Both diagrams can be thought of as cutting a multipole free from figure 4, which corresponds to taking the derivative with respect to that multipole. Accordingly, the field tensors 334, 338 can be considered to be equivalent to the multipole interactions shown on the right hand side of the figure. In other words, field tensor 334 is equivalent to a combination of a $R^{ji(3)}$(333) tensor interacting with a $M^{ji(3)}$(331).$R^{ji(1)}$(327). A similar approach can be applied to field tensor 338, comprising the combined nodes 333, 328: 327.

In order to calculate the angular derivatives of the energy, let $A_{\alpha\beta}$ be rotation matrices which transform vector components from the reference frame to the laboratory frame. Furthermore, suppose that $A_{\alpha\beta}= A_{\alpha\beta}(\phi, \theta, \psi)$, where $(\phi, \theta, \psi)$ are (three) Euler angles and we wish to find the energy derivatives with respect to these angles. Taking the Euler angle $\psi$ as an example, use the chain rule to obtain the contribution from the rank n multipole as:

$$\left( \frac{\partial U}{\partial \psi} \right)_n = \sum_{\alpha\beta\gamma..} \frac{\partial U}{\partial M_{\alpha\beta\gamma..}^{(n)}} \frac{\partial M_{\alpha\gamma..}^{(n)}}{\partial \psi} = \sum_{\alpha\beta\gamma..} \phi_{\alpha\beta\gamma..}^{(n)} K_{\alpha\beta\gamma..}^{\psi(n)} \quad (39)$$

where $()_n$ indicates the contribution from the $n^{th}$ rank multipole and:

$$K_{\alpha\beta\gamma}^{\psi(n)} = n \sum_{\delta\epsilon\zeta..} \frac{\partial}{\partial \psi} \{A_{\alpha\delta}\} A_{\beta\epsilon} A_{\gamma\zeta}.. M_{\delta\epsilon\zeta..}^{ref(n)} \quad (40)$$

where use of index permutation symmetry is made. The total angular derivative is obtained from summing over the contributions from all ranks.

The torques are similarly best worked out in terms of the fields. The torque vector has components , where $\theta_\alpha$ is the angle of rotation about the $\alpha$ axis,, and, by a similar argument to the above, it evaluates to:

$$t_\alpha = - \sum_{n=1}^{\infty} n \sum_{\beta\gamma\delta\epsilon\nu..} \epsilon_{\alpha\beta\gamma} M^{(n)}_{\delta\epsilon\nu..\beta} \phi^{(n)}_{\delta\epsilon\nu..\gamma} \quad (41)$$

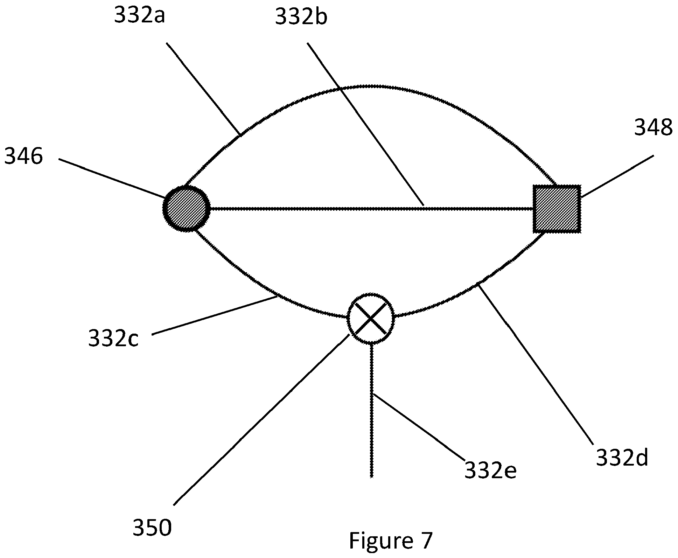

where $\varepsilon_{\alpha\beta\gamma}$ is the Levi-Civita symbol, and where a diagrammatic representation of the torque is given in figure 7. In this figure, interactions between a rank 3 multipole or node 346 and its rank-3 field 348 are represented with two spokes 332a, 332b interconnecting the multipole 346 and field 348. A cross-product 350 is also connected via spokes 332c, 332d. The resultant cross-product is a rank-3 tensor having a further spoke 332e (with its other spokes 332c, 332d being interconnected with the multipole and field respectively).

This section explores the deep connection between homogeneous polynomials and symmetric tensors.

Suppose that $A^{(n)}_{\alpha\beta\gamma..}$ is a symmetric cartesian tensor component where the $\alpha\beta\gamma..$index contains $n_x$ occurrences of $x$, $n_y$ occurrences of $y$ and $n_z$. Defining $\boldsymbol{n} = (n_x, n_y, n_z)$, it will be useful to introduce a compressed tensor notation, in which:

$$A^{(n)}_{\alpha\beta\gamma..} = A^{(n)}_{\beta\alpha\gamma..} = A^{(n)}_{\beta\gamma\alpha..} =.. = A^{(n)}_{(n_x,n_y,n_z)} = A^{(n)}_{\boldsymbol{n}} \quad (42)$$

where, in total, there is a multinomial of permutations of the $\alpha\beta\gamma..$indices belonging to a particular n.

Now suppose that $p^{(n)}(\boldsymbol{r})$ is a homogeneous polynomial of degree n, such that $p^{(n)}(\lambda\boldsymbol{r}) = \lambda^n p^{(n)}(\boldsymbol{r})$, where $\lambda$ is a scalar. For example, a degree 3 homogenous polynomial is given by $p^{a(3)}(\boldsymbol{r}) = 4x^3 + 2y^2x - 5y^3$.

Using compressed notation, it can be shown that $R^{(n)}_{\boldsymbol{n}} = x^{n_x} y^{n_y} z^{n_z}$, and this can label as the polynomial coefficient in $R^{(n)}_{\boldsymbol{n}}$ as $\overline{P}^{(n)}_{\boldsymbol{n}}$. The bar is used to signify that $\overline{P}^{(n)}_{\boldsymbol{n}}$ is a polynomial coefficient, which would otherwise be mistaken as a component of a symmetric tensor. Then, a general degree n homogeneous polynomial can be written as:

$$p^{(n)}(\boldsymbol{r}) = \sum_{|\boldsymbol{n}|=n} \overline{P}^{(n)}_{\boldsymbol{n}} R^{(n)}_{\boldsymbol{n}} = \sum_{\alpha\beta\gamma..} P^{(n)}_{\alpha\beta\gamma..} R^{(n)}_{\alpha\beta\gamma..} \quad (43)$$

where the sum is over all values of $\boldsymbol{n} = (n_x, n_y, n_z)$ for which $n_x + n_y + n_z = n$, and the symmetric tensor, $\boldsymbol{P}^{(n)}$, has been introduced, which has components:

$$P^{(n)}_{\boldsymbol{n}} = \frac{n_x! n_y! n_z!}{n!} \overline{P}^{(n)}_{\boldsymbol{n}} \quad (44)$$

where the inverse multinomial coefficient is required due to the permutation symmetry of the $\alpha\beta\gamma..$indices.

Inner products, $\langle \boldsymbol{P}^{(n)}, \boldsymbol{A}^{(n)} \rangle$, can also be formed, which can be evaluated as:

$$\langle \boldsymbol{P}^{(n)}, \boldsymbol{A}^{(n)} \rangle = \sum_{\alpha\beta\gamma..} P^{(n)}_{\alpha\beta\gamma..} A^{(n)}_{\alpha\beta\gamma..} = \sum_{|\boldsymbol{n}|=n} \overline{P}^{(n)}_{\boldsymbol{n}} A^{(n)}_{\boldsymbol{n}} \quad (44)$$

Given that $\boldsymbol{P}^{(n)}$ encodes all the information in the polynomial coefficients, $\boldsymbol{P}^{(n)}$ can be thought of as being the symmetric tensor form of $\overline{P}^{(n)}_{\boldsymbol{n}}$.

The $\boldsymbol{P}^{(n)}$ tensor may also be obtained from the polynomial itself by way of the gradient operator, according to:

$$\boldsymbol{P}^{(n)} = \frac{1}{n!} \nabla^{(n)} p^{(n)}(\boldsymbol{r}) \quad (45)$$

which may be seen by applying the above gradient operator to equation 42. Using equation 44 the inverse to equation 45 is given by:

$$p^{(n)}(\boldsymbol{r}) = \langle \boldsymbol{P}^{(n)}, \boldsymbol{R}^{(n)} \rangle \quad (46)$$

The linear operator in equation 45 provides a mapping from homogeneous polynomials to their corresponding symmetric tensors. And given the existence of an inverse in equation 46, this mapping is one to one and onto, thus creating an *isomorphism* between the vector spaces of homogeneous polynomials of degree n, and symmetric tensors of rank n. That is, the two vector spaces: (i) the vector space described by symmetric tensors of rank n, and (ii) the vector space described by homogeneous polynomials of degree n are equivalent.

The so-called *harmonic polynomials, $h^{(n)}(\boldsymbol{r})$*, can now be introduced, which are a subset of the $p^{(n)}(\boldsymbol{r})$ polynomials which have a vanishing laplacian, $\Delta\{h^{(n)}(\boldsymbol{r})\} = 0$, where $\Delta = \nabla.\nabla$ is the laplacian operator. As an example, one such polynomial is given by $h^{a(3)} = 2x^3z + 3x^2y - 6xy^2z - y^3$, for which it may be confirmed that $\Delta h^{a(3)} = 0$.

The harmonic polynomials are of particular interest, as their corresponding symmetric tensors, given by , are traceless, due to the vanishing laplacian condition on $h^{(n)}(\boldsymbol{r})$. Thus the linear operator, , creates an isomorphism between the vector spaces of harmonic polynomials of degree n, and symmetric traceless tensors of rank n.

The harmonic polynomials, or equivalently the rank n traceless tensors are spanned by 2n+1 linearly independent vectors. To see this, first consider a rank n symmetric cartesian tensor for which it is a simple matter to show that there are possible values of $\boldsymbol{n} = (n_x, n_y, n_z)$ for which $n_x + n_y + n_z = n$. Furthermore, it is not too difficult to show that for symmetric traceless tensors, the traceless condition imposes constraints, which leaves $N_{st} = N_s - N_t = 2n + 1$ independent components.

For example, it is possible to describe the entire rank-2 tensor $\boldsymbol{M}^{(2)}$ with just $N_{st} = 5$ components. One method is to pick elements $M_{xx}^{(2)}, M_{yy}^{(2)}, M_{xy}^{(2)}, M_{xz}^{(2)}, M_{yz}^{(2)}$, from which we can infer the value of $M_{zz}^{(2)} = -M_{xx}^{(2)} - M_{yy}^{(2)}$ from using the traceless condition, and we can obtain $M_{yx}^{(2)} = M_{xy}^{(2)}$ from use of the permutation symmetry, and likewise for the remaining components.

Given the above, and given the fact that there is an isomorphism between harmonic polynomials of degree n and traceless tensors of rank n it can be deduced that the harmonic polynomials must also have 2n + 1 linearly independent components for degree n.

## Spherical harmonics as a basis for traceless symmetric tensors.

The discussion at the end of the last section referred to the fact that symmetric traceless tensors can in principle be described by a minimal set of 2n+1 linearly independent vectors. It would obviously be advantageous to work in a representation in which just this number of components are used, and in this section will show how this can be done using spherical harmonics, which provide a natural orthonormal basis for traceless tensors.

From the theory of spherical harmonics, an inner product can be defined from the integration of products of harmonic polynomials over the unit sphere according to:

$$\langle h^{a(n)}, h^{b(n)} \rangle\rangle_s = \int h^{a(n)}(\phi, \theta) h^{b(n)}(\phi, \theta) dS \quad (47)$$

where the subscript s notation $\langle,\rangle_s$ is to indicate that this is the spherical inner product, not to be confused with the tensor inner product.

Also from the theory of spherical harmonics, a complete orthogonal basis for the $\langle,\rangle_s$ inner product is provided by the *spherical harmonics* (technically, the regular solid harmonics), which comprise a set of 2n + 1 real harmonic polynomials orthogonal over the unit sphere, such that writing the spherical harmonic polynomials as $q^{i(n)}(\boldsymbol{r})$, and assuming proper normalization, gives:

$$\langle q^{i(n)}, q^{j(n)} \rangle_s = \delta_{ij} \quad (48)$$

where i,j is in the range [0,2n + 1].

Note that a spherical harmonic can be used to describe *any* harmonic polynomial confined to the unit sphere. However, in the present case the term spherical harmonic polynomial refer specifically to the set of $q^{i(n)}(\boldsymbol{r})$ polynomials, which are orthogonal over the unit sphere.

By application of equation 45, the traceless tensor form of the spherical harmonics, $\boldsymbol{Q}^{i(n)}$, can also be defined as :

$$\boldsymbol{Q}^{i(n)} = \frac{1}{n!} \nabla^{(n)} q^{i(n)}(\boldsymbol{r}) \quad (49)$$

which, using equation 46, has an inverse given by:

$$q^{i(n)}(\boldsymbol{r}) = \langle \boldsymbol{Q}^{i(n)}, \boldsymbol{R}^{(n)} \rangle = \sum_{|\boldsymbol{n}|=n} \overline{Q}_{\boldsymbol{n}}^{i(n)} R_{\boldsymbol{n}}^{(n)} \quad (50)$$

Now, given that the vector spaces of (i) harmonic polynomials and (ii) symmetric traceless tensors are isomorphic, and given that their respective inner products are symmetric under any rotation of the axes, such that they can be said to be acting on the tensors themselves, and not just their components in one frame, it is plausible that the two inner products: (i) the tensor inner product written as $\langle,\rangle$, and (ii) the spherical inner product, $\langle,\rangle_s$ defined in equation 47, are also isomorphic. That is, up to trivial rank-dependent normalisation factor, $N^{(n)}$:

$$\langle h^{a(n)}, h^{b(n)} \rangle_s = N^{(n)} \langle \boldsymbol{H}^{a(n)}, \boldsymbol{H}^{b(n)} \rangle \quad (51)$$

where, is the traceless tensor representation of the harmonic polynomial $h^{(n)}(\boldsymbol{r})$.

From the inner product isomorphism, it follows that the $\boldsymbol{Q}^{i(n)}$ symmetric tensors defined in equation 49 form a complete orthogonal basis for traceless tensors of rank n, such that, assuming proper normalization:

$$\langle \boldsymbol{Q}^{i(n)}, \boldsymbol{Q}^{j(n)} \rangle = \delta_{i,j} \quad (52)$$

That $\boldsymbol{Q}^{i(n)}$ provides such a basis allows the expression of any symmetric traceless rank n tensor as a sum in the rank n spherical harmonics according to:

$$\boldsymbol{A}^{(n)} = \sum_{k=1}^{2n+1} A_k^{(n)} \boldsymbol{Q}^{k(n)} \quad (53)$$

and taking the inner product of both sides of equation 53 with respect to $\boldsymbol{Q}^{i(n)}$ shows that $A_i^{(n)}$, the components of $\boldsymbol{A}^{(n)}$ in the spherical harmonic basis are given by:

$$A_i^{(n)} = \langle \boldsymbol{Q}^{i(n)}, \boldsymbol{A}^{(n)} \rangle = \sum_{|\boldsymbol{n}|=n} \overline{Q}_{\boldsymbol{n}}^{i(n)} A_{\boldsymbol{n}}^{(n)} \quad (54)$$

where the spherical harmonic components are indexed by modern roman lower case letters, as opposed to greek for the cartesian indices.

Conversion of traceless tensors from cartesians to spherical harmonics according to equation 54 are perhaps most easily done through consulting tables of spherical harmonics polynomials. And for convenience, the spherical harmonic polynomials up to rank 3 are listed in table I.

As an example, from table I, we have that the spherical harmonic , which gives, , and given that $q^{1(2)} = \left(\sqrt{6}/6\right)(3z^2 - r^2)$, which gives $A_1^{(2)} = \left(\sqrt{6}/6\right)(3A_{zz} - (A_{xx} + A_{yy} + A_{zz}))$, and so on.

The spherical harmonic representation comes in particularly useful for calculating inner products; by using the orthogonality of spherical harmonics to write:

$$\langle \boldsymbol{A}^{(n)}, \boldsymbol{B}^{(n)} \rangle = \sum_{ij=1}^{2n+1} A_i^{(n)} B_j^{(n)} \langle \boldsymbol{Q}^{i(n)}, \boldsymbol{Q}^{j(n)} \rangle = \sum_{i=1}^{2n+1} A_i^{(n)} B_i^{(n)} \quad (55)$$

Thus, it can be seen that calculating an inner product in spherical harmonics requires the minimal 2n + 1 operations for that rank, which is an enormous saving over the $3^n$ multiplications required for naively multiplying all of the $A_{\alpha\beta\gamma..}^{(n)} B_{\alpha\beta\gamma..}^{(n)}$ matrix cartesian components together.

It has now been demonstrated how the $Q_n^{i(n)}$ coefficients allow for transformation of cartesians into spherical harmonics. There is also an inverse transformation, given by $R_k^{\alpha\beta\gamma..(n)}$, which are the components of $R_{\alpha\beta\gamma..}^{(n)}$ projected onto the spherical harmonic basis, and which can be used to transform the components in the spherical harmonic basis back to cartesians.

Table II provides a table convenient way for carrying out these transformations.

As an example, table II gives $xy^2 = -(1/2)q^{6(3)} - (\sqrt{15}/30)q^{2(3)}$, from which it follows that, with spherical harmonic components $A_k^{(3)}$, then . $A_{xyy}^{(3)} = -(1/2)A_6^{(3)} - (\sqrt{15}/30)A_2^{(3)}$

Next we consider a detracing operator.

Suppose that $T^{(n)}$ is an in-general non-traceless symmetric tensor. Given that the spherical harmonics form a complete orthonormal basis for the subspace of traceless rank n tensors, a projection operator $\hat{D}$ can be formed from:

$$\hat{D}T^{(n)} = \sum_{i=1}^{2n+1} \langle T^{(n)}, Q^{i(n)} \rangle Q^{i(n)} \quad (56)$$

where $\hat{D}$ acts to project the tensor into the traceless subspace, such that the result of the above is a traceless tensor. Hence $\hat{D}$ is the detracing operator.

One application of $\hat{D}$ is in particular worth noting. From Hobson's theorem, it can be seen that $S^{(n)}$, the Maxwell Cartesian spherical harmonics from equation 24, are the traceless form of the $R^{(n)}$ tensors, from which it follows that $S^{(n)} = \hat{D}R^{(n)}$. Then, applying the above expression for $\hat{D}R^{(n)}$, and using equation 50 to make the substitution $\langle R^{(n)}, Q^{i(n)} \rangle = q^{i(n)}(r)$ implies that:

$$S_i^{(n)} = q^{i(n)}(r) \quad (57)$$

Thus, the spherical harmonic components of the Maxwell Cartesian spherical harmonics are just the spherical harmonic polynomials themselves.

**The multipole interaction in spherical harmonics**

The aim of this section is to convert the various expressions so far developed in cartesians into their spherical harmonic equivalents.

In the last section it was shown how to convert inner products into spherical harmonics. And in this regard, it's unfortunate the multipole interaction generating formula of equation 31 cannot be expressed entirely in terms of such products, involving as it does problematic contractions of the form $C^{(d_c)} = M^{(d_i+d_c)}.d_i.R^{(d_i)}$.

However, even when dealing with such contractions, there is a way of still using the inner product method, which shall now be described.

The *split-component representation* of a symmetric tensor will now be introduced, using the notation $T^{(n_a,n_b)}$, where $n = n_a + n_b$ is the full rank of the tensor, $T^{(n_a+n_b)}$, of which $T^{(n_a,n_b)}$ is but one representation. Taking $n_a = 3$, $n_b = 2$ as an illustrative example, the symmetric traceless multipole tensor, $M^{(3,2)}$, which has cartesian components can be written as:

$$M_{\alpha\beta\gamma,\delta\epsilon}^{(3,2)} = M_{\alpha\beta\gamma\delta\epsilon}^{(5)} \quad (58)$$

where $M^{(3,2)}$ transforms as a symmetric traceless cartesian tensor with respect to: (i) its before-comma components, (ii) its after-comma components, and (iii) in all its components as a whole. Using this representation, an example contraction can now be written as:

$$C_{\alpha\beta\gamma}^{(3)} = \sum_{\delta\epsilon} M_{\alpha\beta\gamma\delta\epsilon}^{(5)} R_{\delta\epsilon}^{(2)} = \sum_{\delta\epsilon} M_{\alpha\beta\gamma,\delta\epsilon}^{(3,2)} R_{\delta\epsilon}^{(2)} \quad (59)$$

which behaves like an inner product with respect to the after-comma components, and, as such, can be readily evaluated in spherical harmonics.

By separately transforming the before-comma and after-comma components of $M^{(3,2)}_{\alpha\beta\gamma,\delta\epsilon}$ into spherical harmonics, and, using transformations of the sort described by eqn. 43, gives:

$$M^{(3,2)}_{i,j} = \sum_{\alpha\beta\gamma\delta\epsilon} Q^{i(3)}_{\alpha\beta\gamma} Q^{j(2)}_{\delta\epsilon} M^{(5)}_{\alpha\beta\gamma\delta\epsilon} \quad (60)$$

where $Q^{j(2)}_{\delta\epsilon}$ is used to transform $M^{(3,2)}_{\alpha\beta\gamma,\delta\epsilon} \to M^{(3,2)}_{\alpha\beta\gamma,k}$ and $Q^{i(3)}_{\alpha\beta\gamma}$ is used to transform $M^{(3,2)}_{\alpha\beta\gamma,k} \to M^{(3,2)}_{j,k}$. And as discussed in the last section, these transformations are easiest done by way of tables of spherical harmonics, suitably implemented into code.

Also, and referring to the discussion of the detracing operator of equation 56, the $R^{(n)}$ tensor components are transformed as $R^{(n)}_{\alpha\beta} \to S^{(n)}_i = q^{i(n)}(\boldsymbol{r})$, and so the desired contraction can now be written in spherical harmonics as:

$$C^{(3)}_a = \sum_{b=1}^{5} M^{(3,2)}_{a,b} q^{b(2)}(\boldsymbol{r}) \quad (61)$$

Note that The concept of a split component representation can be made quite general. It is possible to use a mixed Cartesian - spherical harmonic representation, such as $M^{(3,2)}_{\alpha\beta\gamma,k}$, or more than one representation can be used; for instance, $M^{(3,2,1)}_{a,b,c}$ is a valid split of $\boldsymbol{M}^{(6)}$. However, no matter how the split is chosen, or the base, it is still referring to the same underlying tensor, and, if necessary, one can always recover all the original components from the by taking the appropriate inverse transformations.

The rank-1 spherical harmonics are just x,y and z (see table I), from which it follows that a rank 1 tensor has spherical harmonic components $T^{(1)}_a = T^{(1)}_x, T^{(1)}_y, T^{(1)}_z$, the same as in cartesians. And, in general, $T^{(1,1,1,..)}_{a,\beta,\gamma,.} = T^{(n)}_{\alpha\beta\gamma..}$.

The split component representation is symmetric with regards to any permutation of its components, e.g. $T^{(m,n)}_{a,b} = T^{(n,m)}_{b,a}$, and $T^{(l,m,n)}_{a,b,c} = T^{(n,l,m)}_{c,a,b} = T^{(n,m,l)}_{c,b,a}$, and so on.

The transformations can all be done by way of the table method explained in the last section. That is, one does not have to carry out tedious matrix multiplications, but can instead just use table I suitably implemented into code to convert the Cartesians into spherical harmonic components.

At this stage it will prove useful to return to the diagrammatic representation.

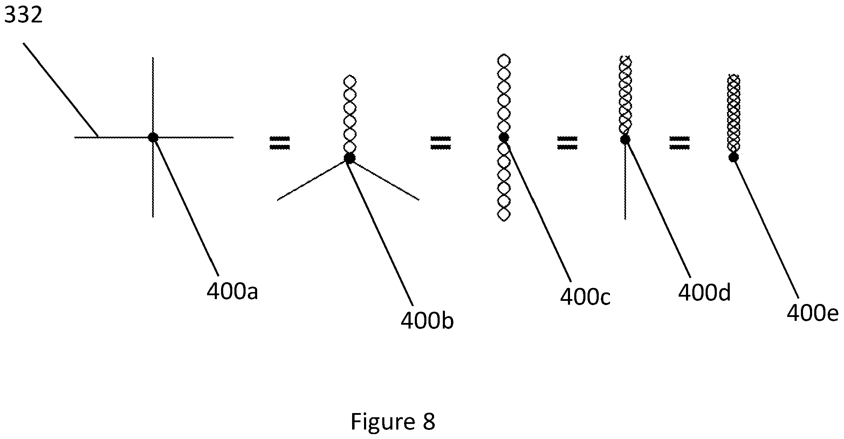

Figure 8 illustrates the equivalence between different representations of the tensors in spherical harmonics and Cartesian coordinates. The example given is of a traceless symmetric rank 4 tensor, shown as a node 400, which can be represented as either $T^{(1,1,1,1)}$(400a), or $T^{(2,1,1)}$(1400b), or $T^{(2,2)}$(400c), or $T^{(3,1)}$(400d), or $T^{(4)}$(400e), where the cartesian coordinates are, as usual, represented by spokes 332, and where the transformation to spherical harmonics is depicted by *braiding* any number of spokes together. Of course, this is just a visual metaphor, but it is intended to convey how the transformation into spherical harmonics intertwines (through taking linear combinations of) multiple Cartesian indices into one spherical harmonic index.

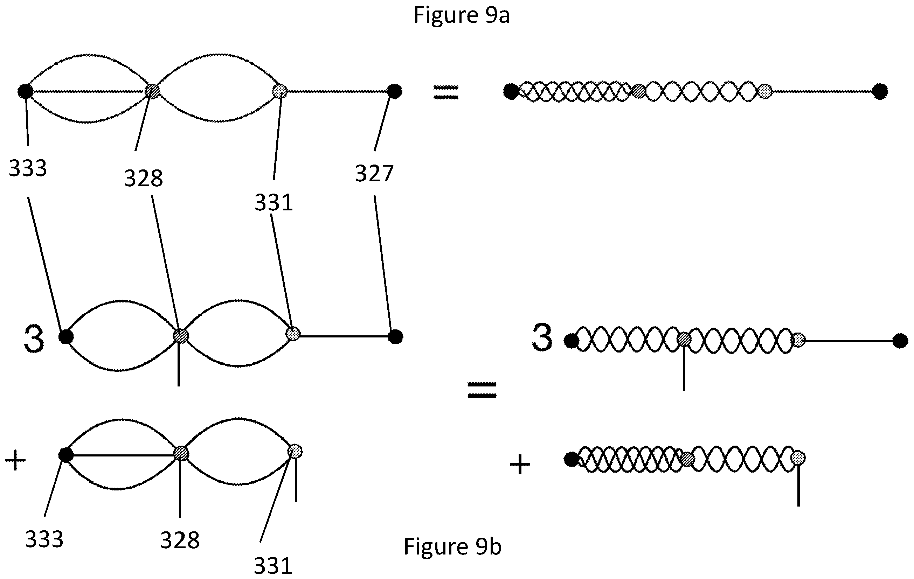

Referring to figure 9a, shows how energy term, ($\boldsymbol{M}^{i(5)}$(328)$\vdots\boldsymbol{R}^{ji(3)}$(333)); ($\boldsymbol{M}^{j(3)}$ (331).$\boldsymbol{R}^{ji(1)}$(327)), depicted in figure 4, can be converted to spherical harmonics, through performing the braidings $M^{i(5)}_{\alpha\beta\gamma\delta\epsilon} \to M^{i(3,2)}_{a,b}$ , $M^{j(3)}_{\alpha\beta\gamma} \to M^{j(2,1)}_{a,b}, R^{ji(2)}_{\alpha\beta} \to q^{a(2)}(\boldsymbol{r}^{ji})$ and $R^{ji(3)}_{\alpha\beta\gamma} \to q^{a(3)}(\boldsymbol{r}^{ji})$, and then calculating the contractions according to

$$\left(\boldsymbol{M}^{i(5)} \vdots \boldsymbol{R}^{ji(3)}\right) : \left(\boldsymbol{M}^{j(3)}. \boldsymbol{R}^{ji(1)}\right) = \sum_{a=1}^{7}\sum_{b=1}^{5} q^{b(3)}(\boldsymbol{r}^{ji}) M^{i(3,2)}_{b,a} \sum_{c=1}^{3} M^{j(2,1)}_{a,c} q^{c(1)}(\boldsymbol{r}^{ji}) \quad (62)$$

And the final diagrammatic equation, in figure 5 bottom, shows how to convert the gradient of the above term into spherical harmonics, which requires the additional braiding $M_{\alpha\beta\gamma\delta\epsilon}^{i(5)} \to M_{a,v,c}^{i(2,1,2)}$.

The methods developed here can be used to transform any contraction, and thus, we are now in a position to transform the entire multipole interaction, energies and forces and fields, into spherical harmonics. We begin with the multipole interaction generating formula of eqn. 31, which in spherical harmonics is given by

$$G_{ji}^l(\boldsymbol{r}^{ji}) = \sum_{d_i+d_c+d_j=l} C_{d_i,d_c,d_j} \sum_{a=1}^{2d_c+12d_i+1} \sum_{b=1} q^{b(d_i)}(\boldsymbol{r}^{ji}) M_{b,a}^{i(d_i,d_c)} \sum_{c=1}^{2d_j+1} M_{a,c}^{j(d_c,d_j)} q^{c(d_j)}(\boldsymbol{r}^{ji}) \quad (63)$$

The spherical harmonic analogue to eqn. 34, which gives the gradient terms necessary for the force calculations (per eqn. 33) is given by

$$\frac{\partial}{\partial r_\gamma^j} G_{ji}^l(\boldsymbol{r}^{ji}) = \sum_{d_i+d_c+d_j=l} C_{d_i,d_c,d_j} \sum_a^{2d_c+1} [d_i \sum_{b=1}^{2d_i-1} q^{b(d_i-1)}(\boldsymbol{r}^{ji}) M_{b,\gamma,a}^{i(d_i-1,1,d_c)} \sum_{c=1}^{2d_j+1} M_{a,c}^{j(d_c,d_j)} q^{c(d_j)}(\boldsymbol{r}^{ji})$$
$$+ d_j \sum_{c=1}^{2d_i+1} q^{c(d_i)}(\boldsymbol{r}^{ji}) M_{c,a}^{i(d_i,d_c)} \sum_{b=1}^{2d_j-1} M_{a,\gamma,b}^{j(d_c,1,d_j-1)} q^{b(d_j-1)}(\boldsymbol{r}^{ji})] \quad (64)$$

and the spherical harmonic analogue to eqn. 25, which gives the derivatives necessary for the multipole fields (per eqn. 24) is given by

$$\frac{\partial}{\partial \boldsymbol{M}^{j(n)}} G_{ji}^l(\boldsymbol{r}^{ji}) = SYMM \left[ \sum_{\substack{d_i+d_j+d_c=l \\ d_c+d_j=n}} C_{d_i,d_c,d_j} \left( \sum_{b=1}^{2d_i+1} q^{b(d_i)}(\boldsymbol{r}^{ji}) M_{b,a}^{i(d_i,d_c)} \right) q^{c(d_j)}(\boldsymbol{r}^{ji}) \right]. \quad (65)$$

This last expression needs some explanation. Each term in the sum has a tensor representation of type $T_{a,c}^{(d_c,d_j)}$, but given that the sum is over $d_c$, $d_j$, the quantity in square brackets will result in a sum in different split component representations, e.g., for rank 4, the sum will have the form $\boldsymbol{S}^{(4)} = c_4 \boldsymbol{T}^{(4)} + c_{3,1} \boldsymbol{T}^{(3,1)} + c_{2,2} \boldsymbol{T}^{(2,2)}$, where the representations do not in general refer to symmetric tensors. However, once the full sum has been evaluated, it can be symmetrised by converting the result back into Cartesians, before averaging over all permutations of the Cartesian indices.

The electrostatic potential at $\boldsymbol{r}$, from a multipole expansion at the origin can be found from the above by taking the rank 0 multipole derivative and then using eqn. 37 to obtain

$$\phi(\boldsymbol{r}) = \sum_{l=0}^{\infty} B_l(r) \sum_{a=1}^{2l+1} q^{a(l)}(\boldsymbol{r}) M_a^{(l)} = \sum_{l=0}^{\infty} B_l(r) \langle \boldsymbol{M}^{(l)}, \boldsymbol{R}^{(l)} \rangle, \quad (66)$$

where the last term can be derived from the first, or obtained from taking the rank 0 multipole derivative of eqn. 38, and where we note that it reduces to eqn. 11 for the kernel

Finally, the spherical harmonic analogue for eqn. 41, giving the total torque on a multipole site is given by

$$t_\alpha = - \sum_{n=1}^{\infty} n \sum_{i=1}^{2n-1} \sum_{\beta\gamma} \epsilon_{\alpha\beta\gamma} M_{i,\beta}^{(n-1,1)} \phi_{i,\gamma}^{(n-1,1)}, \quad (67)$$

Eqns 63-67 then comprise final expressions for the multipole interaction in spherical harmonics, in a form suitable for implementation into the Ewald sum. Here, we should admit that we have not given spherical harmonic equivalents for rotation of the multipoles, or for calculation of the angular derivatives (eqn. 40). It is preferable to keep these in Cartesians for simplicity, but given that both these calculations can be performed outside the main $ij$ particle loop, there is no significant computational cost to their calculation.

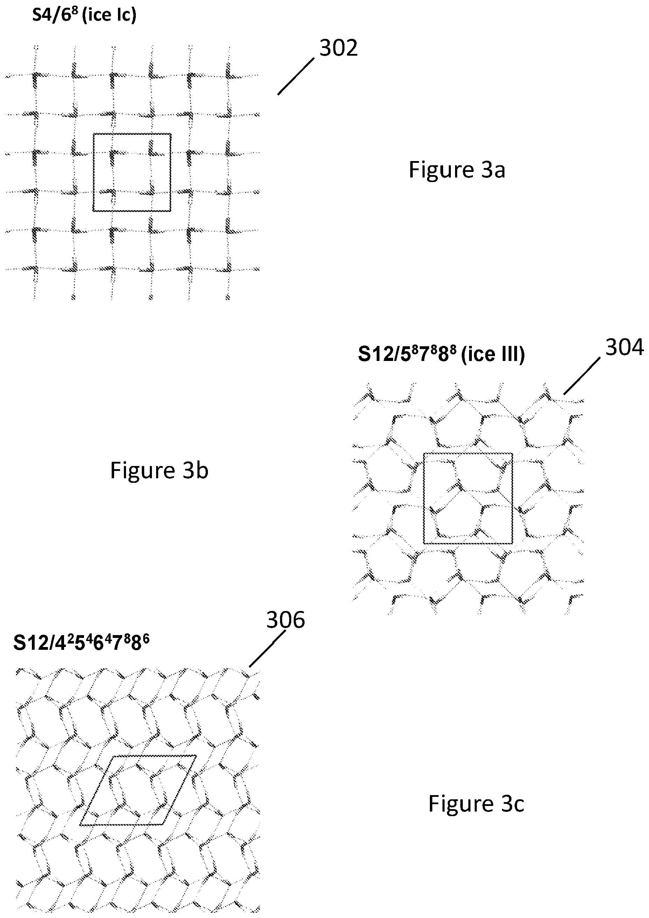

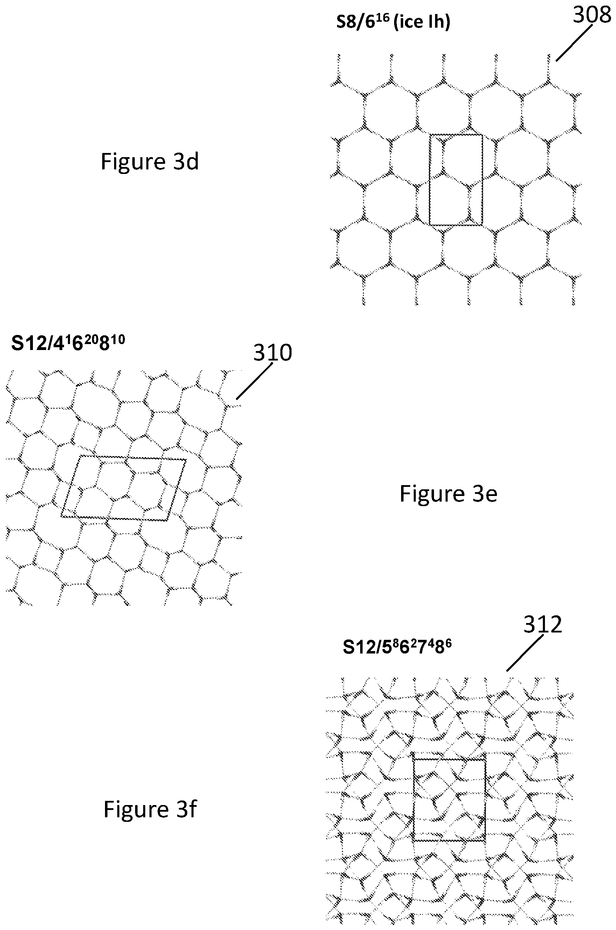

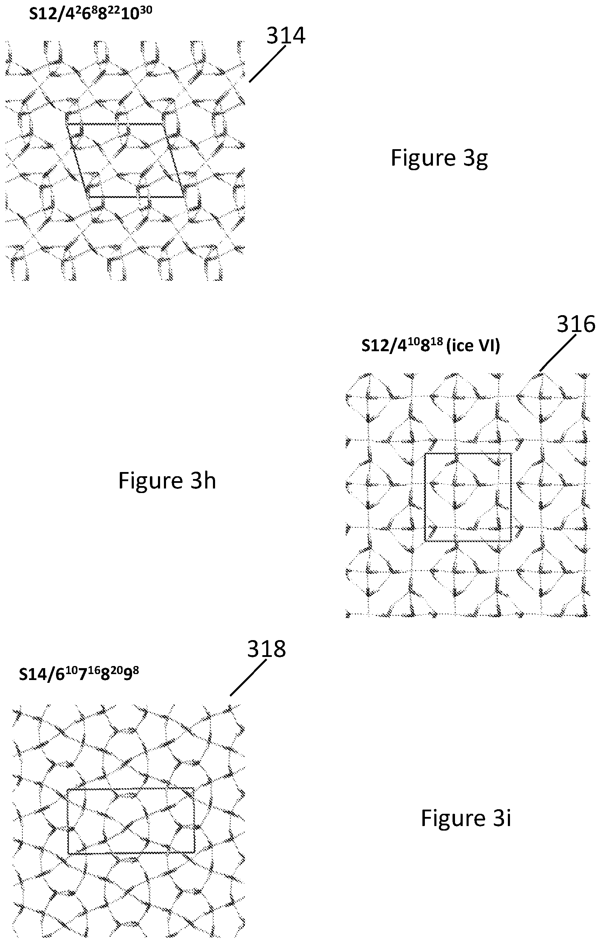

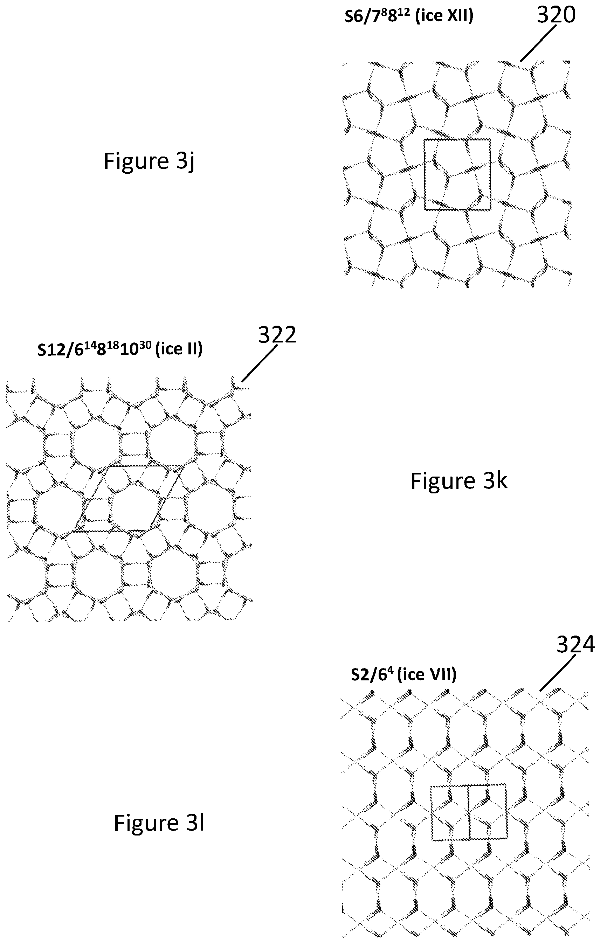

As an example, the supercell method and the crystal structure prediction algorithm was used to predict lowest enthalpy structures of the 4-site TIP4P empirical molecular model of water at various external pressures. The results are shown in Figures 3a to 3l.

The energy for this model is given by summing over all interatomic pairs, where the energy for an *ij* pair is given by

$$u(r_{ij}) = \left(\frac{A_{12}}{r_{ij}^{12}} + \frac{A_6}{r_{ij}^6}\right) + \frac{q_i q_j}{4\pi\epsilon_0 r_{ij}} \quad (68)$$

where $A_{12}$ and $A_6$ are constants. TIP4P is a rigid water model, having a fixed geometry determined by a HOH angle, $\theta_{HOH}$ and OH bond distance, $r_{OH}$. It has a single Lennard Jones interaction site on the oxygen site of each water, charges $q_H$ on the hydrogen atoms, and a charge of $-2q_H$ on a massless 'M-site', which is placed on the molecular bisector, at a distance of $r_{OM}$ from the oxygen nucleus toward the H nuclei. The model parameters are given in table 3.

The electrostatic interactions were handled by implementing an Ewald sum for triclinic periodic cells, where the real space part of the sum together with the Lennard Jones interactions were summed using the supercell technique, which was found to be good enough to converge the energy per simulation cell to about a tenth of a kJ/mol. The reciprocal space part of the sum is unaffected by the supercell method, and was implemented in the standard manner.

To prevent discontinuities at the cut-off, the Lennard Jones pair interactions were multiplied by a sigmoidal cubic smoothstep function, S(x), which smoothly interpolates from $S(x) = 1; x < 0.9r_c$ to $S(x) = 0; x > r_c$. With this unction implemented, it's appropriate to modify the expression for $U^{LR}$, the long-range energy (equation 2) such that the integral is from $0.95\ r_c$ to $\infty$.

The real space part of the Ewald sum involves summing over Gaussian charge distributions of the form , and the reciprocal space part of the sum involves summing over its Fourier transform pair: , where $\varepsilon$ is a freely chosen parameter, which determines the convergence of the Ewald sum. To ensure good convergence, we want $g(r)$ to be very small at the real space cut-off, and we will also choose a cut-off in reciprocal space, $k_c$, such that $G(k)$ is similarly small at $k_c$. To this end, we choose $\zeta$ and $k_c$ such that

$$\epsilon = g(r_c) = G(k_c) \quad (69)$$

where $\delta$ is a very small number. In the present case $\delta = 10^{-7}$.

Inverting the above gives:

$$\zeta = -\sqrt{log(\epsilon)}/r_c \quad (70)$$

and

$$k_c = 2\zeta^2 r_c \quad (71)$$

The calculations were all performed using a custom in-house code, but the energies were checked against a standard molecular dynamics package, to make sure that the results are correct and reproducible.

Local optimisations were done using the FRPRMN subroutine of Numerical Recipes[1] which is an implementation of the Fletcher-Reeves conjugate gradient algorithm.

The basin-hopping implementation used steps drawn from Gaussian distributions with sigma values of $\sigma_f = 0.08$ for each fractional centre of mass coordinate, $\sigma_e = 1$ radians for each Euler angle, and $\sigma_c = 0.05$ Å for each of the six independent cell vector components. Each Monte Carlo step consisted of first choosing a molecule at random, and then performing a random translation and rotation for that molecule, coupled with a random displacement in all six cell vector components. A temperature of 250 K was found to be near optimal for the Metropolis Monte Carlo acceptance criterion.

Locating global minima is a hard problem, even with a good algorithm. To stand the best chance of actually finding the global minimum, for each case multiple independent basin-escape simulations were used, each starting from different random initial configurations, and using different random-number seeds for the Monte Carlo displacements. In total, a population of 24 independent basin-escape trajectories was used for each structure, which were each run on separate cores. This acts to effectively parallelise the problem, and also to increase diversity in the search, diversity being a key concern given that individual walks can be highly correlated over long times, as they can get trapped in funnels of low-lying minima.

Another advantage of using independent trajectories is that it gives some assurance that the putative global minima are likely correct, if the same lowest minimum structure is found from multiple trajectories, begun from different initial conditions.

In the present case, a range of the best (i.e. lowest enthalpy) structures were calculated, where the best candidates hopefully include the true global minimum structure. To achieve this, every minimum accepted during the course of each quasi Monte Carlo walk is stored to form a list of candidate minima, which can later be sorted and ranked.

Example structures are shown in Figures 3a to 3l. Each structure 302-324 represents a possible crystal structure for ice. As described in further detail below, each structure comprises a series of Hydrogen bonds (H-bonds) that help to orientate the water molecules in a number of alternative ice polymorphs.

Many of the ice polymorphs are proton disordered. That is, for a given hydrogen-bond configuration, the position of the protons can adopt any number of possible configurations, while still obeying the ice rules. With this in mind, the present work is interested in finding the best (i.e. lowest enthalpy) structures only up to a proton ordering, such that, only the lowest enthalpy structure found for each different type of hydrogen-bond network is counted.

For each minimum-energy structure a list of H-bonds is created, where two water molecules are considered hydrogen-bonded if their OO distance is less than 3.5 Å, and the H-O - - H angle is less than 30 degrees.

The task then is to know whether two structures share the same hydrogen-bond network. This is a non-trivial task, essentially equivalent to the graph-theory problem of deciding the isomorphism of two graphs. A (partial) solution was to perform a ring-analysis of each minimum energy structure, in which a list of water tetramer, pentamer, hexamer etc. rings present in the structure is created. Each ring is a hydrogen-bonded circuit of water molecules, where only those rings which cannot be further decomposed into smaller rings are listed.

Note, this is not a full solution because it is possible that two structures could have the same number of molecules per unit cell, and the same ring-decomposition, and yet have different hydrogen-bonding networks. Nevertheless, this was the method in the present case.

This ring-decomposition allows one to develop a naming scheme, in which the structures are labelled by their number of rings and the number of molecules per unit cell. In this scheme, the structure S $12/5^86^47^88^8$ - 312, for example, has 12 (the number following the 'S') molecules per unit cell. The numbers following the forward slash give the ring count, and this structure has eight five-membered rings, four six-membered rings, eight seven-membered rings and eight eight-membered rings. In each of the figures shown in Figures 3a to 3l, a box shows the unit cell, which is then used as the simulation cell according to the method described above.

In the present case, ring decompositions up to ten-membered rings are calculated.

A systematic search was performed search in the range 1-14 TIP4P water molecules in periodic boundary conditions, and at pressures: 0 bar, 4000 bar and 8000 bar.

The search located hundreds of structures, but tables 4a, 4b and 4c list the ten best (lowest-enthalpy) structures found at each pressure, a selection of the best structures is shown in figures 3a to 3l, and the coordinates for all the structures is supplied in supplementary information.

Turning to table 4a, at zero pressure the best structure is tied between an ice Ic and an ice Ih structure (structures S4/6$^8$ 302 and S8/6$^{16}$ 308), which have (to this precision) identical energies of -57.104 kJ/mol (per molecule).

Note that ice lattices with even smaller unit cells were also found. The crystal structure prediction code did locate an ice Ic lattice using just a two-molecule simulation cell, and an ice Ih lattice with just a four-molecule simulation cell. However, both of these were higher in energy than could be found with the larger cells, presumably due to the better proton ordering afforded by the larger unit cells.

The third best structure found was an ice III structure, S12/5$^8$7$^8$8$^8$ 304, which is just 0.3 kJ/mol higher in energy than the best ice Ih and Ice Ic structures.

The ordering of the structures completely changes at 4000 bar, and looking at table 2b, at this higher pressure, the ice Ih and ice Ic structures are no longer even in the best ten, and an ice III structure 304 is now the one with the lowest enthalpy. Outside of the top ten, an ice VI structure, S10/4$^{10}$8$^{18}$ 316 was also located.

Finally, at 8000 bar the ice III structure 304 is still in first place, but the crystal structure prediction algorithm also located in third place, an ice XII structure, S6/7$^8$8$^{12}$ 320, just behind S12/4$^2$6$^8$8$^2$10$^{30}$ 314, which doesn't appear to be one of the known polymorphs. Outside of the top ten, an ice VII structure, S2/6$^4$ 324 was also located, which is a high pressure phase consisting of two interpenetrating ice Ic lattices.

Two zero-pressure crystalline structures were found possessing the lowest energy per molecule: an ice Ic structure 302, with a smallest unit cell of four molecules, and an ice Ih structure 308, with a smallest unit cell of eight molecules, with both structures having near identical energies (to five significant figures).

That the energies of ice Ic 302 and ice Ih 308 are very close is not too much of a surprise, given that they are both ice structures with a practically identical tetrahedrally coordinated first-neighbour shell around each molecule. However, it is surprising that the best structures found appear to have practically identical energies. However, the structures do differ (however slightly) in their densities, so it is suspected that the agreement in their energies is the result of a numerical coincidence, and not for any deeper physical reason. Indeed, it was found that slightly varying the model charges or applying an external pressure is enough to break the agreement.

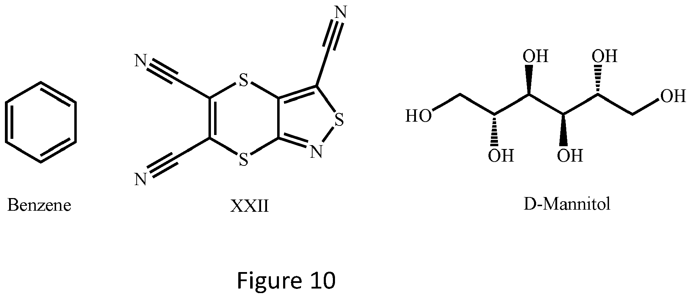

In the following, the crystal structure of three different organic molecules (viz., Benzene, Molecule XXII and D-Mannitol, shown in Figure 10) is predicted using the basin-hopping algorithm.

The methodology considers the following four steps:

*1. Exploration of the conformational preferences of the target molecules*

For benzene there is only one conformer, whereas, for molecule XXII, we observed two conformers which are mirror image of each other. For mannitol, all possible molecular conformations of the molecule are explored using ab-initio molecular dynamics simulations of a single molecule along a trajectory of 50 ps. Then equally spaced 2500 geometries are considered - each geometry using B3LYP/6-31G(d) level of theory. 104 conformers for mannitol are distinguished considering their optimized energies and normal modes. Thus, a list of library of possible conformers of the target molecule is produced.

*2. Development of Force-Field Parameters*

An orthogonal box with 4 molecules is considered (the volume of the box was approximated using an *ab-initio* NPT simulation) and then, NVT simulation was done for 50 ps which takes around a month with 192 CPU. The resulting trajectory was then used to fit an empirical potential energy surface in which the total intermolecular potential energy is defined to be the sum of (i) electrostatic terms, which are handled using atomic multipole interactions up to octopole-octopole, and (ii) pairwise additive interatomic interactions which are modelled using radial functions in an inverse power series of the form $U = A/r^4 + B/r^8 + C/r^{10}$, where r is the interatomic separation, and A, B and C are parameters.

The parameters are determined by using a variant of the 'force-matching' algorithm, in which the parameters are optimized to best reproduce the DFT force on the centre of mass force of each molecule and the DFT torque about the centre of mass over the course of the trajectory. The total intermolecular energy of the system is also taken into account. The philosophy here is to only fit to properties which are insensitive to intramolecular contributions, which the forces on the centre of mass, and the torque about those centre of masses are.

The interaction between different atoms is in general very different, for instance, the interaction between a H and an O atom will be very different to that between two H's, or two O's, and so separate A, B, C parameters must be chosen for each different interaction type. It is found that even the interactions between the same atom types are highly dependent on their local chemical environment. For this reason, the interactions are distinguished by not just the types of atom involved in the pair, but also by the atoms each member of the pair is bonded to. So, for example, a H bonded to an O is counted as a different type to a H bonded to a C. The resulting number of different interaction types is quite large, such that typically the order of ~100 distinct radial functions are dealt with to model the intermolecular interactions of a 30 atoms, which in turn means there are -300 parameters to fit. Force matching though can handle this, and can find all these parameters in a matter of minutes.

As mentioned above, the electrostatic interactions are evaluated using the multipole method. In this method, a multipole expansion of the atomic charge distributions for molecules in the gas phase is calculated using the GDMA software (version 2.2.11), which finds atomic multipole coefficients describing the electrostatic potential arising from the electronic structure charge distribution, giving a much superior description to traditional point charge models. The electrostatic component of the intermolecular energies with the empirical model are then calculated by summing the electrostatic interactions between each multipole, using expressions from the theory of multipoles. In this present invention, this sum was extended up to the octopole-octopole level, which should give a very accurate description of the electrostatics.

*3. Generating Plausible Crystal-Packing Arrangements of the Target Molecules*

The basin hoppling method disclosed in the present invention is used to explore possible crystal packing arrangements. To summarise, once the empirical model has been constructed, the problem is to find the best crystal structures with that empirical potential energy surface. This is done by essentially searching for minimum energy configurations in periodic boundary conditions and finding the minima with the lowest energies. This turns out to be a difficult challenge, because the number of possible minima may be enormous, and so a 'global optimization' algorithm needs to be used which has a chance of locating the lowest energy minima in a reasonable time. The so-called 'Basin-Hopping Monte-Carlo' global optimization algorithm is used here.

The standard Monte-Carlo algorithm performs a random walk over configuration space, accepting or rejecting moves based on their energy difference with respect to the previous step, which can be shown to produce the correct thermal distribution of structures in the long time limit.

The Basin-Hopping Monte-Carlo algorithm is a modification of the original Monte-Carlo algorithm in which the accept/reject step is made with respect to energies of the local minima at each point in the walk. That is, before a new structure is accepted and rejected, the system is relaxed via an optimization algorithm to its local minimum, and it is the relative energy of the minima which is used to determine whether a new structure is accepted or not as the next structure in the Monte Carlo 'walk'. The introduction of a local optimization at each step in the Monte Carlo slows down the walk, by maybe an order of magnitude, but it is also found to produce walks which are much better at overcoming barriers between minima, and hence is much better at locating low-lying minima.

### 4. Conformer Searching

So far only the intermolecular interactions are discussed, but real molecules are flexible, and this flexibility has to be taken into account if the correct crystal structured are to be predicted. In this current study a conformer-based approach is used. First a library was constructed of gas-phase conformer geometries, corresponding to local minima on the DFT surface for single molecules. Then the Basin-Hopping Monte-Carlo simulation, which in the present implementation treats the molecules as rigid, is allowed to 'flip' between different conformers during the course of each walk. That is, the geometry of each molecule in the simulation cell is always set to be equal to one of the conformers in the conformer library, but Monte Carlo moves are introduced in which each molecule has a chance of flipping to a different conformer, with the probability as usual determined by the energy difference of the (relaxed) periodic structures before and after the flip has been effected.

### 5. Ranking the Resulting Predicted Crystal Structure

The predicted crystal structure is re-ranked using high-level DFT calculations. The 50 lowest energy crystals predicted using Basin Hoppling method are considered for each molecule. Note that rigid molecules in the Basin Hopping method are considered. Both the atomic positions and lattice vectors are relaxed using dispersion-corrected DFT method. This relaxation improved the reliability of our predicted crystals.

## Computational Details

Ab-initio molecular-dynamics simulations (both NPT and NVT) were performed using Density Functional Theory (DFT) with Perdew-Burke-Ernzerhof (PBE)[1] functional and GPW formalism, as implemented in the QUICKSTEP module of CP2K package. Norm-conserving Goedecker-Teter-Hutter (GTH) pseudopotentials were used along with the double-zeta valence polarized (DZVP) basis sets to expand the Kohn-Sham valence orbital with an energy cutoff of 320 Ry with periodic boundary conditions. The noncovalent van der Waals interactions were considered using the Grimme (DFT-D3) method.

To explore all the possible molecular conformations of the molecule, ab-initio molecular dynamics simulations of a single molecule was carried out along a trajectory of 50 ps. Equally spaced 2500 geometries are considered and optimized each geometry using B3LYP/6-31G(d) level of theory. The optimized geometries are distinguished considering their optimized energies and normal modes.

For ranking of the predicted crystals, the geometries were reoptimised along with latticed relaxation using DFT with plane-wave basis with plane-wave energy cutoff of 400 eV and general gradient approximation (GGA) for exchange-correlation energy functional in the version of Perdew, Burke and Emzerhof (PBE).

## Results and Discussion

### 1. Exploring Plausible Conformations of the target molecules

For benzene there is only one conformer, whereas, for molecule XXII, two conformers were observed which are mirror image of each other. For mannitol, all possible molecular conformations of the molecule were explored using ab-initio molecular dynamics simulations of a single molecule along a trajectory of 50 ps. Equally spaced 2500 structures were considered from the 50 ps ab initio trajectory and optimized each structure using B3LYP/6-31G(d) level of theory.

### 2. Ranking the Resulting Predicted Crystal Structure

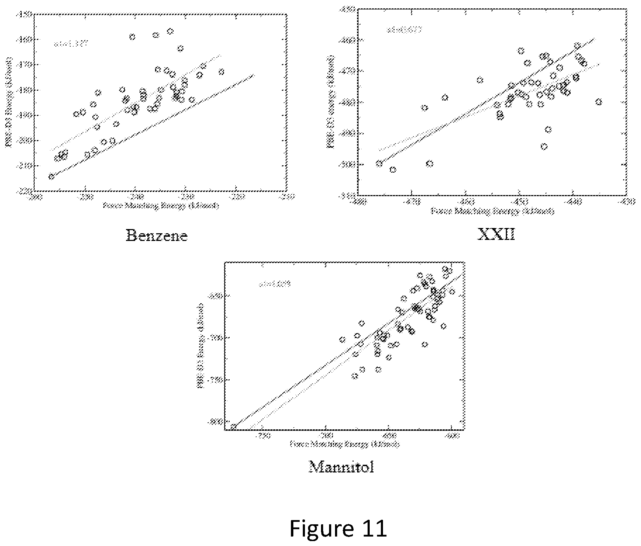

Independent Basin Hopping Monte Carlo runs are performed in parallel on multiple processors to generate tens of thousands of putative crystal structure minima. The results are then filtered to find the lowest lying minima. The predicted crystal structure is then ranked using high level DFT calculations, i.e. the best structures as predicted are taken from Basin Hopping Monte Carlo, and then re-relax these structures under the full DFT potential energy surface. The 50 lowest energy crystals predicted using Basin Hoppling Monte Carlo method for each molecules are considered. In figure 11, the DFT energies are plotted against force-matching energy below.

*3. Comparison Between Predicted Crystal Structure and Experimental Crystal Structure*

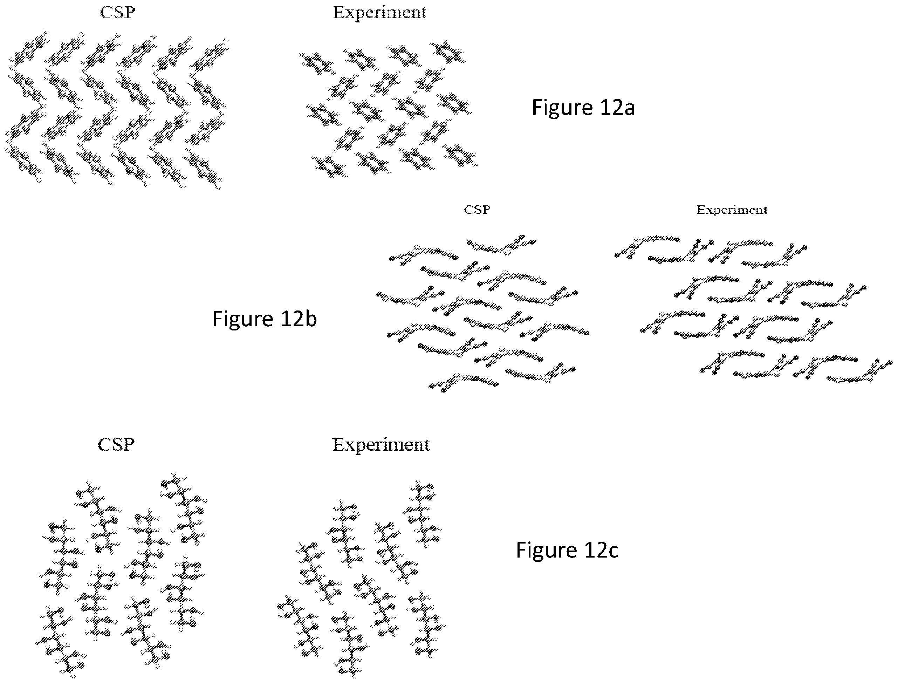

The predicted crystal structures for benzene, XXII and D-Mannitol are compared against the experimentally-predicted structure in figures 12a-c. The predicted crystals are well comparable with the experimental crystals, with space-group equivalency.

**Table I. Spherical harmonics $q^{i(n)}(r)$ in cartesians up to rank 3.**

The spherical harmonics are normalised such that $\| \boldsymbol{Q}^{i(n)} \| = 1$, where $\boldsymbol{Q}^{i(n)}$ are the tensor forms of the $q^{i(n)}(r)$ polynomials.

Rank 0

$q^{i(0)} = 1$

Rank 1

$q^{1(1)} = x$ $\qquad$ $q^{2(1)} = y$ $\qquad$ $q^{3(1)} = z$

Rank 2

$$q^{1(2)} = \left(\sqrt{6}/6\right)(3z^2 - r^2) \qquad q^{2(2)} = \left(\sqrt{2}\right)xz \qquad q^{3(2)} = \left(\sqrt{2}\right)yz,$$

$$q^{4(2)} = \left(\sqrt{2}/2\right)(x^2 - y^2) \qquad q^{5(2)} = \left(\sqrt{2}\right)xy$$

Rank 3

$$q^{2(3)} = \left(\sqrt{15}/10\right)x(5z^2 - r^2)$$

$$q^{3(3)} = \left(\sqrt{15}/10\right)y(5z^2 - r^2)$$

$$q^{5(3)} = \sqrt{6}xyz$$

$$q^{6(3)} = (1/2)x(x^2 - 3y^2)$$

$$q^{7(3)} = (1/2)y(3x^2 - y^2)$$

**Table II. Cartesians in terms of spherical harmonics, up to rank 3**

Rank 2

$$x^2 = -\left(\sqrt{6}/6\right)q^{1(2)} + \left(\sqrt{2}/2\right)q^{4(2)} \qquad y^2 = -\left(\sqrt{6}/6\right)q^{1(2)} - \left(\sqrt{2}/2\right)q^{4(2)}$$

$$z^2 = \left(\sqrt{6}/3\right)q^{2(2)} \qquad xy = \left(\sqrt{2}/2\right)q^{5(2)}$$

$$xz = \left(\sqrt{2}/2\right)q^{2(2)} \qquad yz = \left(\sqrt{2}/2\right)q^{3(2)}$$

Rank 3

$$x^3 = (1/2)q^{6(3)} - \left(\sqrt{15}/10\right)q^{2(3)} \qquad x^2y = (1/2)q^{7(3)} - \left(\sqrt{15}/30\right)q^{3(3)}$$

$$xy^2 = -(1/2)q^{6(3)} - \left(\sqrt{15}/30\right)q^{2(3)} \qquad y^3 = -(1/2)q^{7(3)} - \left(\sqrt{15}/10\right)q^{3(3)}$$

$$x^2z = \left(\sqrt{6}/6\right)q^{4(3)} - \left(\sqrt{10}/10\right)q^{1(3)} \qquad xyz = \left(\sqrt{6}/6\right)q^{5(3)}$$

$$y^2z = -\left(\sqrt{6}/6\right)q^{4(3)} - \left(\sqrt{10}/10\right)q^{1(3)} \qquad xz^2 = 2\left(\sqrt{15}/15\right)q^{2(3)}$$

$$yz^2 = 2\left(\sqrt{15}/15\right)q^{3(3)} \qquad z^3 = \left(\sqrt{2}/5\right)q^{1(3)}$$

Table 3: Model parameters of the TIP4P model of water. Also, note the reported densities were calculated using the (slightly idealised) atomic masses $m_H = m_P$, $m_O = 16.0$ $m_P$ and $m_M = 0$, where $m_P$ is the proton mass: $m_P = 1.67262178 * 10^{-27}$ kg.

| | |
|---|---|
| $q_H$ ($|e|$) | 0.52 |
| $q_M$ ($|e|$) | -1.04 |
| $A_{12}$ (kJ/mol A$^{12}$) | 2510400.00 |
| $A_6$ (kJ/mol A$^6$) | -2552.24 |
| $\theta_{HOH}$ (degrees) | 104.52 |
| $r_{OH}$ (A) | 0.9572 |
| $r_{OM}$ (A) | 0.15 |

Table 4a: The ten lowest enthalpy per molecule structures at 0 bar, together with their enthalpies and densities.

| Structure | enthalpy (kJ/mol) | density (g/cm$^3$) |
|---|---|---|
| S4/6$^8$ (ice Ic) – 302 | -57.104 | 0.9851 |
| S8/6$^{16}$ (ice Ih) – 308 | -57.104 | 0.9835 |
| S12/5$^8$7$^8$8$^8$ (ice III) – 304 | -56.793 | 1.2508 |
| S12/4$^1$6$^{20}$8$^{10}$ - 310 | -56.647 | 0.9660 |
| S14/5$^2$6$^{22}$7$^2$8$^6$- 312 | -56.593 | 0.9840 |
| S14/6$^{28}$8$^4$ | -56.581 | 1.0046 |

| Structure | enthalpy (kJ/mol) | density (g/cm$^3$) |
|---|---|---|
| S12/5$^8$6$^2$7$^4$8$^6$ – 312 | -56.580 | 1.2366 |
| S12/5$^8$6$^4$7$^8$8$^8$ | -56.578 | 0.9535 |
| S12/4$^2$5$^4$6$^4$7$^8$8$^6$- 306 | -56.577 | 1.2362 |
| S12/5$^2$6$^{16}$7$^8$ | -56.557 | 0.9924 |

Table 4b. The ten lowest enthalpy per molecule structures at 4000 bar. Also shown, an ice VI structure, $S10/4^{10}8^{18}$.

| Structure | enthalpy (kJ/mol) | density (g/cm$^3$) |
|---|---|---|
| $S12/5^87^88^8$ (ice III) - 304 | -51.086 | 1.2895 |
| $S12/4^25^46^47^88^6$- 306 | -50.818 | 1.2800 |
| $S12/5^86^27^48^6$- 312 | -50.802 | 1.2725 |
| $S12/4^26^88^{22}10^{30}$- 314 | -50.672 | 1.3586 |
| $S12/6^{14}8^{18}10^{30}$ (ice II) - 322 | -50.609 | 1.2969 |
| $S12/5^66^67^48^89^4$ | -50.548 | 1.3300 |
| $S6/7^88^{12}$- 320 | -50.394 | 1.4020 |
| $S14/4^45^66^48^69^410^{24}$ | -50.380 | 1.3727 |
| $S14/5^76^17^98^{10}9^610^1$ | -50.362 | 1.3402 |
| $S10/5^46^27^48^{16}$ | -50.342 | 1.3758 |
| ... | … | … |
| $S10/4^{10}8^{18}$ (ice VI) - 316 | -49.989 | 1.4529 |

Table 4c. The ten lowest enthalpy per molecule structures at 8000 bar. Also shown, an ice VII structure, $S2/6^4$.

| Structure | enthalpy (kJ/mol) | density (g/cm$^3$) |
|---|---|---|
| $S12/5^87^88^8$ (ice III) - 304 | -45.533 | 1.3220 |
| $S12/4^26^88^{22}10^{30}$- 314 | -45.380 | 1.3817 |
| $S6/7^88^{12}$ (ice XII) - 320 | -45.261 | 1.4234 |
| $S14/6^{10}7^{16}8^{20}9^8$- 318 | -45.256 | 1.4434 |
| $S12/4^25^46^47^88^6$- 306 | -45.227 | 1.3132 |

| Structure | enthalpy (kJ/mol) | density (g/cm³) |
|---|---|---|
| $S12/5^6 6^6 7^4 8^8 9^4$ | -45.172 | 1.3671 |
| $S12/5^8 6^2 7^4 8^6$ | -45.169 | 1.3018 |
| $S14/4^4 5^6 6^4 8^6 9^4 10^{24}$ | -45.143 | 1.3970 |
| $S10/5^4 6^2 7^4 8^{16}$ | -45.114 | 1.3983 |
| $S8/4^2 7^4 8^{20} 9^4$ | -45.112 | 1.4383 |
| ... | ... | ... |
| $S2/6^4$ (ice VII) − 324 | -39.691 | 1.6157 |

In additional or alternative embodiments, as well as optimising the degrees of freedom describing the position of each molecule, for crystal structure prediction with empirical model potentials it is also useful to take into account molecular flexibility.

Even if the intramolecular degrees of freedom are neglected, there can be an interplay between the intra and intermolecular geometries, such that the large scale crystal structure may depend on how the geometries of the individual molecules can distort.

Unfortunately, it is incredibly difficult to devise intramolecular potentials which can accurately describe how individual molecules can distort. However, one can resort to approximations. The primary approximation used in the present embodiment is to represent the entire intramolecular potential energy surface by its local minima, that is by the geometries and energies of the possible local minima structures of each individual molecule.

The general strategy is as follows: first construct a representative database of local minima for a molecule of interest, recording the energies and geometries of those minima. Then, find some way of hopping from one minimum to another through 'conformation space'. This can be done as part of the larger crystal structure prediction algorithm described above, such that the algorithm is optimising not just in the usual degrees of freedom describing the position of each molecule, but also in the conformation space of each molecule, in the hope of finding a crystal structure which is the minimum energy configuration for all possible arrangements and all possible conformers of the constituent molecules.

The first task then is to obtain a library of conformer minima (and their associated geometries). This is done by running a density functional theory (DFT) trajectory of the molecule in the gas phase, from which optimisations are spawn (at fixed length intervals, e.g. every 100 steps). The resulting structures are sorted by energy and diagonal moments of inertia to remove duplicates.

The next task is to find a way of identifying similar conformers, which helps define paths through conformer space that are most efficient to walk through. This is done through calculation of the relative mean square displacement (MSD) of the nuclei.

Let the MSD between two conformers m and n be given by:

$$\mathrm{MSD}_{m,n} = \frac{\sum_i w_i |r_i^m - r_i^n|^2}{\sum_i w_i} \quad (72)$$

where $r_i^m$ is the cartesian coordinate of the ith nucleus in the mth conformer. The $w_i$ are weight factors which can be set to unity, or which can be chosen to assign different importance to different nuclei. In the present case, $w_i = m_i$, where $m_i$ is the mass of the ith nucleus.

Since both the m and n coordinates describe rigid molecule geometries, the MSD is a function of three translational degrees of freedom and three rotational degrees of freedom. One can eliminate the three translational degrees of freedom by insisting that they are both placed such that their centre of mass coincides with the origin. Then, only three rotational degrees of freedom are left, which can be described by three Euler angles, $\phi$, $\theta$ and $\psi$. To do this, one can define:

$$r_i^m = R(\phi, \theta, \psi) s_i^m \quad (73)$$

where $s_i^n$ can be thought of as the body-centred coordinates of conformer n, and where $R(\phi, \theta, \psi)$ is a 3x3 orthogonal rotation matrix defined by the Euler angles. Because only the relative orientation is important, one only needs to consider rotation of one of the pair, and so $s_i^n = r_i^n$. Thus, the MSD is given by:

$$\mathrm{MSD}_{m,n}(\phi, \theta, \psi) = \frac{\sum_i w_i |R(\phi, \theta, \psi) s_i^m - s_i^n|^2}{\sum_i w_i} \quad (74)$$

To find out which Euler angles minimise the total MSD, a conjugate gradient algorithm is used, which will require the angular derivatives with respect to the Euler angles. These are given by:

$$\frac{\partial}{\partial \phi}\{\mathrm{MSD}_{m,n}(\phi, \theta, \psi)\} = \frac{2}{\sum_i w_i}\sum_i w_i \frac{\partial R(\phi, \theta, \psi)}{\partial \phi} \cdot (R(\phi, \theta, \psi) s_i^m - s_i^n) \quad (75)$$

and similarly for $\theta$ and $\psi$.

The result of the conjugate gradient optimisations is that for each m-n pair, one can find the minimum MSD with respect to the Euler angles as:

$$\min[\mathrm{MSD}_{m,n}] = \mu_{m,n} \quad (76)$$

where $\mu_{m,n}$ are matrix elements with associated Euler angles $\phi_{m,n}^{min}, \theta_{m,n}^{min}, \psi_{m,n}^{min}$.

The $\mu_{m,n}$ matrix elements tell us what the minimum MSD is between each pair of conformers, but one still needs a rule for how to step between conformers. The approach in the present case is to define a stochastic matrix with elements $P_{m,n}$ which gives the probability of flipping from the conformer $m \to n$ on a conformer flip step, where the $P_{m,n}$ are to be some function of the $\mu_{m,n}$ matrix elements, in such a way that the probability is greater to flip between conformers which have smaller MSDs. There is no unique way of doing this, however, in the present case the method was to choose:

$$P_{m,n} = \frac{exp(-\beta \mu_{m,n})}{\sum_k exp(-\beta \mu_{m,k})} \quad (77)$$

where $\beta$ is a freely chosen parameter, that acts like an inverse temperature, and where it can be verified that the probabilities sum to unity:

$$\sum_n P_{m,n} = 1 \quad (78)$$

When $\beta \to 0$ (high temperture), the probabilities equalise, with a step just as likely to visit one conformer as any other conformer, and as $\beta \to \infty$ (i.e. the temperature goes to zero), steps are only possible to the conformer which has the lowest MSD with respect to the starting conformer.

In practice, $\beta$ should be chosen such that steps involving low relative MSDs are favoured, but where the fictional temperature is high enough such that the system will eventually visit every conformer with a reasonable probability. The coordinates of a particular conformer during the course of a simulation can be represented as three COM coordinates, and three rational coordinates, once again given by Euler angles, which arel labelled as $(\phi^0, \theta^0, \psi^0)$. Now, suppose that a particular conformer is allowed to flip from $m \to n$, and we want to update its Euler angles such that it undergoes the minimal MSD change before and after the flip. This can be done by first finding the rotation matrix $R^0 = R(\phi^0, \theta^0, \psi^0)$, then finding the rotation matrix $R_{m,n}^{min} = R(\phi_{m,n}^{min}, \theta_{m,n}^{min}, \psi_{m,n}^{min})$, and then finding the compound rotation matrix $R = R_{m,n}^{min} R^0$, which can then be inverted to obtain the new values of $(\phi^0, \theta^0, \psi^0)$.

The conformer search algorithm is easily incorporated into a basin-hopping monte-carlo type algorithm. This is done by randomly choosing a certain percentage of the monte-carlo steps to involve picking a molecule at random, and then flipping the conformer of that molecule to another conformer, according to the probabilities in the stochastic matrix, which have been worked out beforehand.

The earlier method for building up a database of the conformers is not guaranteed to be exhaustive. However, it may be possible to iterate the entire process of conformer search followed by crystal structure prediction in such a way that the right conformers are (eventually) likely to be found. This can be done by first building an initial database of conformers, which are then used for a crystal structure prediction run. The resulting best structures from this run can then be relaxed under DFT, to produce new conformers that can be augmented to the conformer database, in preparation for more crystal structure prediction steps, and so on.

**Claims**

1. A method of determining one or more stable crystal structures of a system (200), each crystal structure comprising a repeated unit cell, by calculating a configurational energy of the system (200) with periodic boundary conditions, the configurational energy resulting from non-electrostatic interactions and electrostatic interactions, the system (200) having a plurality of particles (212), wherein the particles (212) comprise atoms and molecules, said method comprising the computer-implemented steps (i) to (ix) of:

   i) defining a cut-off radius, said radius defining a non-electrostatic interaction potential cut-off distance between the particles (212) in the system (200);
   ii) defining a set of cell vectors to generate a simulation cell (210);
   iii) defining a set of supercell vectors (230) to generate a supercell, wherein said supercell comprises a plurality of replicas of the simulation cell (210);
   iv) calculating, for any one of the particles (212) located within the supercell, non-electrostatic pair potentials between the one of the particles (212) and first surrounding ones of the particles (212) located from the one of the particles (212) within a boundary (220) defined by the cut-off radius, said non-electrostatic pair potentials resulting from the interaction between the one of the particles (212) with the first surrounding ones of the particles (212), wherein the first surrounding ones of the particles (212) include ones of particles (212) lying outside the supercell (210);
   v) summing the calculated non-electrostatic pair potentials for the particles (212) located within the supercell to provide a non-electrostatic configurational energy of the system;
   vi) accounting for electrostatic interactions, wherein each particle (212) comprises one or more particle multipoles and the electrostatic interactions between the particles (212) comprise multipole interactions between the corresponding one or more particle multipoles that are combined using an Ewald sum;
   vii) calculating a global minimum by dividing the calculated configurational energy of the system by a total number of simulation cells (210) to define a potential energy per simulation cell (210); and
   viii) determining the global minimum of the potential energy per simulation cell (210) by obtaining, by gradient descent, a plurality of local minima of the configurational energy, and by selecting, using a basin-hopping global optimisation algorithm, the lowest one of the local minima to be the global minimum, and wherein the unit cell is equivalent to the simulation cell (210); and
   ix) selecting the crystal structure having the global minimum to be a candidate for the stable crystal structure;

   the method further comprising:
   determining potential properties of the selected stable crystal structure and accordingly controlling the necessary parameters for manufacturing the stable crystal structure

2. The method of claim 1, wherein the step iv) considers additional image particles surrounding a particle using the standard minimum image convention to select said non-electrostatic pair potentials resulting from the interaction of the particle with closest particles or image particles located inside and/or outside the supercell.

3. The method of claim 1 or claim 2, wherein the calculating step iv) comprises the steps of:
   calculating, for any one of the particles (212) located within a selected simulation cell (210), non-electrostatic pair potentials between the one of the particles (212) and second surrounding ones of the particles (212) surrounding the one of the particles (212) within the boundary of the cut-off radius; and wherein the summing step v) comprises the steps of:

summing the calculated non-electrostatic pair potentials calculated for the particles (212) located within the selected simulation cell (210); and

multiplying the summation by the number of the simulation cell (210) inside the supercell, to obtain configurational energy, and wherein the configurational energy is a non-electrostatic potential energy per supercell of the system (200).

4. The method of claim 1 to 3, further comprising a step of converting the multipole interactions from a Cartesian coordinate system into a spherical harmonic form suitable for implementation into the Ewald sum;

diagrammatically representing the multipole interactions as a series of nodes (326, 327, 328, 330, 333, 334, 338, 346, 400) and spokes (332) radiating from the nodes (326, 327, 328, 330, 333, 334, 338, 346, 400), wherein each node represents a symmetric multipole tensor defining a multipole of the particle (212), and wherein the number of spokes (332) radiating from each node (326, 327, 328, 330, 333, 334, 338, 346, 400) is equal to a rank of the symmetric multipole tensor; and

conjoining spokes of interacting multipoles to form spoked connections between the respective nodes, each spoked connection representing an interaction between the nodes.

5. The method of claim 4, wherein the symmetric multipole tensors are traceless.

6. The method of claims 1 to 5, wherein the basin-hopping global optimisation algorithm comprises the steps of:

a) obtaining a local minimum of $H(\mathbf{X})$ with coordinate $\mathbf{X}$ by employing a local optimisation algorithm;

b) generating a random displacement vector $\Delta\mathbf{X}$ to obtain new trial coordinates $\mathbf{X^{trial}} = \mathbf{X} + \Delta\mathbf{X}$;

c) accepting $\mathbf{X^{trial}}$ if $\mathbf{X^{trial}}$ produces separation between any two of the particles (212) inside the simulation cell (210) greater than or equal to a set minimum distance $r^{min}$, or rejecting $\mathbf{X^{trial}}$ rejecting if separation between any two of the particles (210) inside the supercell is smaller than $r^{min}$;

d) repeating steps b) to c) until an acceptable value of $\mathbf{X^{trial}}$ is found;

e) calculating a transformed enthalpy $H^{min}(\mathbf{X^{trial}})$ per simulation cell (210) by employing the local optimisation algorithm on $H(\mathbf{X^{trial}})$;

f) setting $H = H^{min}$ and $\mathbf{X} = \mathbf{X^{trial}}$ if the standard Metropolis Monte Carlo criterion is met: $R < \min [1, exp (-\beta(H^{min}(\mathbf{X^{trial}}) - H(\mathbf{X})))]$, where $R$ is a uniform random number between 0 and 1, and, where $k_B$ is Boltzmann's constant; otherwise, rejecting $H^{min}(\mathbf{X^{trial}})$ and returning $\mathbf{X}$ to the value of the last accepted local minimum before the rejection; and

g) repeating steps b) and f) to calculate a plurality of local minima of $H(\mathbf{X})$.

7. The method of claim 6, step c), wherein $r^{min}$ is constrained by a set maximum

8. The method of claim 7, wherein $H(\mathbf{X})$ comprises contributions from intramolecular potential energy corresponding to a conformer of a molecule of the particles (212) inside the simulation cell

9. The method of claim 8, wherein in step g), calculating the plurality of local minima of $H(\mathbf{X})$ comprises taking into account different ones of the conformer of the molecule from a conformer database, each conformer corresponding to a local minimum of an intramolecular potential energy surface of the molecule.

10. The method of claim 9, wherein in step g), calculating the plurality of local minima of $H(\mathbf{X})$ further comprises calculating a transition probability, said transition probability indicating the probability of the molecule transitioning from a first one of the conformer to a second one of the conformer at a given temperature

11. The method of any one of claim 1 to 10 wherein the size of the supercell is varied during the basin-hopping global optimisation algorithm such that the supercell is always large enough to hold the cut-off radius

12. The method of any one of the above claims, wherein the one or more stable crystal structures are candidates for a pharmaceutical product.

**Patentansprüche**

1. Verfahren zum Ermitteln einer oder mehrerer stabiler Kristallstrukturen eines Systems (200), jede Kristallstruktur umfassend eine wiederholte Einheitszelle, durch Berechnen einer Konfigurationsenergie des Systems (200) mit periodischen Randbedingungen, wobei die Konfigurationsenergie aus nicht-elektrostatischen Wechselwirkungen und elektrostatischen Wechselwirkungen resultiert, wobei das System (200) eine Vielzahl von Partikeln (212) aufweist, wobei die Partikel (212) Atome und Moleküle umfassen, wobei das Verfahren die computerimplementierten Schritte (i) bis (ix) umfasst:

> i) Definieren eines Cut-off-Radius, wobei der Radius eine nicht-elektrostatische Wechselwirkungspotential-Cut-off-Entfernung zwischen den Partikeln (212) in dem System (200) definiert;
> ii) Definieren eines Satzes von Zellvektoren, um eine Simulationszelle (210) zu erzeugen;
> iii) Definieren eines Satzes von Superzellvektoren (230), um eine Superzelle zu erzeugen, wobei die Superzelle eine Vielzahl von Replikaten der Simulationszelle (210) umfasst;
> iv) Berechnen, für ein beliebiges von den innerhalb der Superzelle befindlichen Partikeln (212), von nicht-elektrostatischen Paarpotentialen zwischen dem einen beliebigen von den Partikeln (212) und ersten umgebenden von den Partikeln (212), befindlich ausgehend von dem einen beliebigen von den Partikeln (212) innerhalb einer durch den Cut-off-Radius definierten Grenze (220), wobei die nicht-elektrostatischen Paarpotentiale aus der Wechselwirkung zwischen dem einen beliebigen von den Partikeln (212) mit den ersten umgebenden von den Partikeln (212) resultieren, wobei die ersten umgebenden von den Partikeln (212) außerhalb der Superzelle (210) liegende von den Partikeln (212) beinhalten;
> v) Summieren der berechneten nicht-elektrostatischen Paarpotentiale für die innerhalb der Superzelle befindlichen Partikel (212), um eine nicht-elektrostatische Konfigurationsenergie des Systems bereitzustellen;
> vi) Berücksichtigen von elektrostatischen Wechselwirkungen, wobei jedes Partikel (212) einen oder mehrere Partikelmultipole umfasst und die elektrostatischen Wechselwirkungen zwischen den Partikeln (212) Multipolwechselwirkungen zwischen dem entsprechenden einen oder den entsprechenden mehreren Partikelmultipolen umfassen, die unter Verwendung einer Ewald-Summe kombiniert werden;
> vii) Berechnen eines globalen Minimums durch Teilen der berechneten Konfigurationsenergie des Systems durch eine Gesamtzahl von Simulationszellen (210), um eine Potentialenergie pro Simulationszelle (210) zu definieren; und
> viii) Ermitteln des globalen Minimums der Potentialenergie pro Simulationszelle (210) durch Erhalten, durch Gradientenabstieg, einer Vielzahl von lokalen Minima der Konfigurationsenergie, und durch Auswählen, unter Verwendung eines globalen basin-hopping Optimierungsalgorithmus, des niedrigsten der lokalen Minima als das globale Minimum, und wobei die Einheitszelle äquivalent zu der Simulationszelle (210) ist; und
> ix) Auswählen der Kristallstruktur mit dem globalen Minimum als einen Kandidaten für die stabile Kristallstruktur;

wobei das Verfahren weiter umfasst:
Ermitteln von potentiellen Eigenschaften der ausgewählten stabilen Kristallstruktur, und entsprechendes Steuern der notwendigen Parameter zum Herstellen der stabilen Kristallstruktur.

**2.** Verfahren nach Anspruch 1, bei welchem der Schritt iv) zusätzliche Bildpartikel, die ein Partikel umgeben, unter Verwendung der Standard-Minimum-Bildkonvention berücksichtigt, um die nicht-elektrostatischen Paarpotentiale auszuwählen, die aus der Wechselwirkung des Partikels mit nächstgelegenen Partikeln oder Bildpartikeln resultieren, die innerhalb und/oder außerhalb der Superzelle befindlich sind.

**3.** Verfahren nach Anspruch 1 oder Anspruch 2, bei welchem der Berechnungsschritt iv) die Schritte umfasst:
Berechnen, für ein beliebiges von den innerhalb einer ausgewählten Simulationszelle (210) befindlichen Partikeln (212), von nicht-elektrostatischen Paarpotentialen zwischen dem einen beliebigen von den Partikeln (212) und zweiten umgebenden von den Partikeln (212), die das eine beliebige von den Partikeln (212) innerhalb der Grenze des Cut-off-Radius umgeben; und wobei der Summierungsschritt v) die Schritte umfasst:

> Summieren der berechneten nicht-elektrostatischen Paarpotentiale, die für die innerhalb der ausgewählten Simulationszelle (210) befindlichen Partikel (212) berechnet wurden; und
> Multiplizieren der Summierung mit der Anzahl der Simulationszelle (210) innerhalb der Superzelle, um eine Konfigurationsenergie zu erhalten, und wobei die Konfigurationsenergie eine nicht-elektrostatische potentielle Energie pro Superzelle des Systems (200) ist.

**4.** Verfahren nach Anspruch 1 bis 3, weiter umfassend einen Schritt des Umwandelns der Multipolwechselwirkungen von einem kartesischen Koordinatensystem in eine sphärische harmonische Form, die zur Implementierung in die Ewald-Summe geeignet ist;

schematisches Darstellen der Multipolwechselwirkungen als eine Reihe von Knoten (326, 327, 328, 330, 333, 334, 338, 346, 400) und von den Knoten (326, 327, 328, 330, 333, 334, 338, 346, 400) ausgehenden Speichen (332), wobei jeder Knoten einen symmetrischen Multipoltensor darstellt, der einen Multipol des Partikels (212) definiert, und wobei die Anzahl der von jedem Knoten (326, 327, 328, 330, 333, 334, 338, 346, 400) ausgehenden Speichen (332) gleich einem Rang des symmetrischen Multipoltensors ist; und

Verbinden von Speichen interagierender Multipole, um Speichenverbindungen zwischen den jeweiligen Knoten zu bilden, wobei jede Speichenverbindung eine Wechselwirkung zwischen den Knoten darstellt.

5. Verfahren nach Anspruch 4, bei welchem die symmetrischen Multipoltensoren spurlos sind.

6. Verfahren nach Anspruch 1 bis 5, bei welchem der globale basin-hopping Optimierungsalgorithmus die Schritte umfasst:

a) Erhalten eines lokalen Minimums von $H(\mathbf{X})$ mit der Koordinate $\mathbf{X}$ unter Verwendung eines lokalen Optimierungsalgorithmus;

b) Erzeugen eines Zufallsverschiebungsvektors $\Delta\mathbf{X}$, um neue Versuchskoordinaten $\mathbf{X}^{trial} = \mathbf{X} + \Delta\mathbf{X}$ zu erhalten;

c) Akzeptieren von $\mathbf{X}^{trial}$, wenn $\mathbf{X}^{trial}$ eine Trennung zwischen beliebigen zwei von den Partikeln (212) innerhalb der Simulationszelle (210) erzeugt, die größer oder gleich einer eingestellten Mindestentfernung $r^{min}$ ist, oder Ablehnen von $\mathbf{X}^{trial}$, wenn die Trennung zwischen beliebigen zwei von den Partikeln (210) innerhalb der Superzelle kleiner als $r^{min}$ ist;

d) Wiederholen der Schritte b) bis c), bis ein akzeptabler Wert von Xtrial gefunden ist;

e) Berechnen einer transformierten Enthalpie $H^{min}(\mathbf{X}^{trial})$ pro Simulationszelle (210) unter Verwendung des lokalen Optimierungsalgorithmus auf $H(\mathbf{X}^{trial})$;

f) Einstellen von $H = H^{min}$ and $\mathbf{X} = \mathbf{X}^{trial}$ wenn das Standard Metropolis Monte Carlo Kriterium erfüllt ist: $R < min$ [1, $exp$ (-$\beta$($H^{min}(\mathbf{X}^{trial})$ - $H(\mathbf{X})$))], wobei R eine gleichmäßige Zufallszahl zwischen 0 und 1 ist,, und , wobei $k_B$ die Boltzmann-Konstante ist; andernfalls Ablehnen von $H^{min}(\mathbf{X}^{trial})$ und Zurücksetzen von $\mathbf{X}$ auf den Wert des letzten akzeptierten lokalen Minimums vor der Ablehnung; und

g) Wiederholen der Schritte b) und f), um eine Mehrzahl von lokalen Minima von $H(\mathbf{X})$ zu berechnen.

7. Verfahren nach Anspruch 6, Schritt c), wobei $r^{min}$ durch ein festgelegtes Maximum beschränkt ist.

8. Verfahren nach Anspruch 7, bei welchem H(X) Beiträge von intramolekularer potentieller Energie umfasst, die einem Konformer eines Moleküls der Partikel (212) innerhalb der Simulationszelle entsprechen.

9. Verfahren nach Anspruch 8, bei welchem in Schritt g) das Berechnen der Mehrzahl von lokalen Minima von H(X) das Berücksichtigen verschiedener Konformer des Moleküls aus einer Konformerdatenbank umfasst, wobei jeder Konformer einem lokalen Minimum einer Oberfläche intramolekularer potentieller Energie des Moleküls entspricht.

10. Verfahren nach Anspruch 9, bei welchem in Schritt g) das Berechnen der Mehrzahl von lokalen Minima von H(X) weiter das Berechnen einer Übergangswahrscheinlichkeit umfasst, wobei die Übergangswahrscheinlichkeit die Wahrscheinlichkeit angibt, dass das Molekül bei einer gegebenen Temperatur von einem ersten Konformer zu einem zweiten Konformer übergeht.

11. Verfahren nach einem der Ansprüche 1 bis 10, bei welchem die Größe der Superzelle während des globalen Basin-Hopping Optimierungsalgorithmus variiert wird, damit die Superzelle immer groß genug ist, um den Cut-off-Radius zu halten.

12. Verfahren nach einem der obigen Ansprüche, bei welchem die eine oder mehreren stabilen Kristallstrukturen Kandidaten für ein pharmazeutisches Produkt sind.

**Revendications**

1. Procédé de détermination d'une ou plusieurs structures cristallines stables d'un système (200), chaque structure cristalline comprenant une cellule unitaire répétée, en calculant une énergie de configuration du système (200) avec des conditions limites périodiques, l'énergie de configuration résultant d'interactions non électrostatiques et d'interactions électrostatiques, le système (200) ayant une pluralité de particules (212), dans lequel les particules (212) comprennent des atomes et des molécules, ledit procédé comprenant les étapes (i) à (ix) mises en oeuvre par ordinateur de :

i) définir un rayon de coupure, ledit rayon définissant une distance de coupure de potentiel d'interaction non électrostatique entre les particules (212) dans le système (200) ;

ii) définir un ensemble de vecteurs de cellule pour générer une cellule de simulation (210) ;

iii) définir un ensemble de vecteurs de supercellule (230) pour générer une supercellule, dans lequel ladite supercellule comprend une pluralité de répliques de la cellule de simulation (210) ;

iv) calculer, pour une particule quelconque parmi les particules (212) située dans la supercellule, des potentiels de paire non électrostatiques entre la une particule quelconque parmi les particules (212) et des premières particules environnantes parmi les particules (212) situées à partir de la une particule quelconque parmi les particules (212) à l'intérieur d'une limite (220) définie par le rayon de coupure, lesdits potentiels de paire non électrostatiques résultant de l'interaction entre la une particule quelconque parmi les particules (212) et les premières particules environnantes parmi les particules (212), dans lequel les premières particules environnantes parmi les particules (212) comprennent des particules parmi les particules (212) situées à l'extérieur de la supercellule (210) ;

v) additionner les potentiels de paire non électrostatiques calculés pour les particules (212) situées à l'intérieur de la supercellule afin d'obtenir une énergie de configuration non électrostatique du système ;

vi) prendre en compte des interactions électrostatiques, chaque particule (212) comprenant un ou plusieurs multipôles de particules et les interactions électrostatiques entre les particules (212) comprenant des interactions multipolaires entre le ou les multipôles de particules correspondants qui sont combinés à l'aide d'une somme d'Ewald ;

vii) calculer un minimum global en divisant l'énergie de configuration calculée du système par un nombre total de cellules de simulation (210) pour définir une énergie potentielle par cellule de simulation (210) ; et

viii) déterminer le minimum global de l'énergie potentielle par cellule de simulation (210) en obtenant, par descente de gradient, une pluralité de minima locaux de l'énergie de configuration, et en sélectionnant, à l'aide d'un algorithme d'optimisation globale à saut de bassin, le plus bas des minima locaux comme étant le minimum global, et dans lequel la cellule unitaire est équivalente à la cellule de simulation (210) ; et

ix) sélectionner la structure cristalline présentant le minimum global comme candidate à la structure cristalline stable ;

le procédé comprenant en outre

déterminer des propriétés potentielles de la structure cristalline stable sélectionnée et contrôler en conséquence les paramètres nécessaires à la fabrication de la structure cristalline stable.

2. Procédé selon la revendication 1, dans lequel l'étape iv) prend en compte des particules image supplémentaires entourant une particule en utilisant la convention d'image minimale standard pour sélectionner lesdits potentiels de paire non électrostatiques résultant de l'interaction de la particule avec des particules les plus proches ou des particules image situées à l'intérieur et/ou à l'extérieur de la supercellule.

3. Procédé selon la revendication 1 ou la revendication 2, dans lequel l'étape de calcul iv) comprend les étapes de : calculer, pour une particule quelconque parmi les particules (212) située dans une cellule de simulation sélectionnée (210), des potentiels de paires non électrostatiques entre la une particule quelconque parmi les particules (212) et des deuxièmes particules environnantes parmi les particules (212) entourant la une particule quelconque parmi les particules (212) à l'intérieur de la limite du rayon de coupure ; et dans lequel l'étape d'addition v) comprend les étapes de :

additionner les potentiels de paire non électrostatiques calculés pour les particules (212) situées dans la cellule de simulation sélectionnée (210) ; et

multiplier la somme par le nombre de cellules de simulation (210) à l'intérieur de la supercellule, pour obtenir une énergie de configuration, et dans laquelle l'énergie de configuration est une énergie potentielle non électrostatique par supercellule du système (200)

4. Procédé selon l'une des revendications 1 à 3, comprenant en outre une étape de convertir des interactions multipolaires depuis un système de coordonnées cartésiennes en une forme harmonique sphérique adaptée à la mise en oeuvre de la somme d'Ewald ;

représenter schématiquement des interactions multipolaires sous la forme d'une série de noeuds (326, 327, 328, 330, 333, 334, 338, 346, 400) et de rayons (332) rayonnant à partir des noeuds (326, 327, 328, 330, 333, 334, 338, 346, 400), dans lequel chaque noeud représente un tenseur multipolaire symétrique définissant un multipôle de la particule (212), et dans lequel le nombre de rayons (332) rayonnant à partir de chaque noeud (326, 327, 328, 330, 333, 334, 338, 346, 400) est égal à un rang du tenseur multipolaire symétrique ; et joindre des rayons de multipôles en interaction pour former des connexions en rayons entre les noeuds respectifs, chaque connexion en rayons représentant une interaction entre les noeuds.

5. Procédé selon la revendication 4, dans lequel les tenseurs multipolaires symétriques sont sans trace.

6. Procédé selon les revendications 1 à 5, dans lequel l'algorithme d'optimisation globale à saut de bassin comprend les étapes suivantes :

a) obtenir un minimum local de $H(\mathbf{X})$ avec une coordonnée $\mathbf{X}$ en employant un algorithme d'optimisation local ;
b) générer un vecteur de déplacement aléatoire $\Delta\mathbf{X}$ pour obtenir des nouvelles coordonnées de test $\mathbf{X^{trial}} = \mathbf{X} + \Delta\mathbf{X}$;
c) accepter $\mathbf{X^{trial}}$ si $\mathbf{X^{trial}}$ produit une séparation entre deux particules quelconques parmi les particules (212) à l'intérieur de la cellule de simulation (210) supérieure ou égale à une distance minimale fixée $r^{min}$, ou rejeter $\mathbf{X^{trial}}$ si la séparation entre deux particules quelconques parmi les particules (212) à l'intérieur de la supercellule est inférieure à $r^{min}$ ;
d) répéter les étapes b) à c) jusqu'à ce qu'une valeur acceptable de $\mathbf{X^{trial}}$ soit trouvée ;
e) calculer une enthalpie transformée $H^{min}(\mathbf{X^{trial}})$ par cellule de simulation (210) en utilisant l'algorithme d'optimisation local sur $H(\mathbf{X^{trial}})$ ;
f) fixer $H = H^{min}$ et $\mathbf{X} = \mathbf{X^{trial}}$ si le critère standard de Metropolis Monte Carlo est respecté : $R <$ min [1, $exp$ $(-\beta(H^{min}(\mathbf{X^{trial}})-H(\mathbf{X})))$], où $R$ est un nombre aléatoire uniforme entre 0 et 1, et , où $k_B$ est la constante de Boltzmann; sinon, rejeter $H^{min}(\mathbf{X^{trial}})$ et renvoyer $\mathbf{X}$ à la valeur du dernier minimum local accepté avant le rejet; et
g) répéter les étapes b) et f) pour calculer une pluralité de minima locaux de $H(\mathbf{X})$.

7. Procédé selon la revendication 6, étape c), dans lequel $r^{min}$ est limité par un maximum fixé.

8. Procédé selon la revendication 7, dans lequel H(X) comprend des contributions d'énergie potentielle intramoléculaire correspondant à un conformère d'une molécule des particules (212) à l'intérieur de la cellule de simulation.

9. Procédé selon la revendication 8, dans lequel à l'étape g), le calcul de la pluralité de minima locaux de $H(\mathbf{X})$ comprend la prise en compte de différents conformères parmi les conformères de la molécule à partir d'une base de données de conformères, chaque conformère correspondant à un minimum local d'une surface d'énergie potentielle intramoléculaire de la molécule.

10. Procédé selon la revendication 9, dans lequel, à l'étape g), le calcul de la pluralité de minima locaux de $H(\mathbf{X})$ comprend en outre le calcul d'une probabilité de transition, ladite probabilité de transition indiquant la probabilité que la molécule passe d'un premier conformère à un deuxième conformère à une température donnée.

11. Procédé selon l'une quelconque des revendications 1 à 10, dans lequel la taille de la supercellule est variée au cours de l'algorithme d'optimisation globale par saut de bassin, de sorte que la supercellule est toujours suffisamment grande pour maintenir le rayon de coupure.

12. Procédé selon l'une quelconque des revendications précédentes, dans lequel la ou les structures cristallines stables sont des candidates pour un produit pharmaceutique.

Figure 1

Figure 2

**S4/6⁸ (ice Ic)**

302

Figure 3a

**S12/5⁸7⁸8⁸ (ice III)**

304

Figure 3b

**S12/4²5⁴6⁴7⁸8⁶**

306

Figure 3c

S8/6[16] (ice Ih)

308

Figure 3d

S12/4[1]6[20]8[10]

310

Figure 3e

S12/5[8]6[2]7[4]8[6]

312

Figure 3f

$S12/4^2 6^8 8^{22} 10^{30}$

314

Figure 3g

$S12/4^{10} 8^{18}$ (ice VI)

316

Figure 3h

318

$S14/6^{10} 7^{16} 8^{20} 9^8$

Figure 3i

S6/7⁸8¹² (ice XII)   320

Figure 3j

S12/6¹⁴8¹⁸10³⁰ (ice II)   322

Figure 3k

S2/6⁴ (ice VII)   324

Figure 3l

Figure 4

Figure 5a

Figure 5b

Figure 6

EP 3 948 877 B1

Figure 7

EP 3 948 877 B1

332

400a

400b

400c

400d

400e

Figure 8

Figure 9a

333

328

331

327

3

+

333

328

331

3

+

EP 3 948 877 B1

Figure 9b

Benzene            XXII            D-Mannitol

Figure 10

Benzene

XXII

Mannitol

Figure 11

CSP Experiment

Figure 12a

CSP Experiment

Figure 12b

CSP Experiment

Figure 12c

EP 3 948 877 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PODESZWA et al.** *Phys. Chem. Chem. Phys.,* 2009, vol. 11, 5512-5518